# DEMANDE DE BREVET EUROPEEN

(11) **EP 1 250 919 A1**
(43) Date de publication de la demande: **23.10.2002**
(21) Numéro de dépôt: 02290882.6
(22) Date de dépôt: 09.04.2002
(51) Int. Cl.: A61K 7/50

(54) **Compositions de bain gelifiantes, relaxantes**

(30) Priorité: 13.04.2001 FR 0105112
(71) Demandeur: L'OREAL, 75008 Paris (FR)
(72) Inventeur: L'Alloret, Florence, 75013 Paris (FR); Tournilhac, Florence, 75011 Paris (FR)
(74) Mandataire: Audier, Philippe André

(57) **Abrégé**

Composition de bain gélifiante relaxante, comprenant au moins un polymère ou composé gélifiant, ledit polymère ou composé gélifiant étant un polymère ou composé thermogélifiant dont les propriétés de gélification par chauffage sont réversibles avec la température.

Utilisation de ce polymère ou composé pour gélifier un volume ou une masse d'eau à une température déterminée dans un récipient, et fluidifier ledit volume d'eau lors de son refroidissement, afin de faciliter l'évacuation dudit récipient, tel qu'une baignoire.

## Description

La présente invention a trait à des compositions de bain gélifiantes, relaxantes, éventuellement moussantes.

L'invention a également trait à l'utilisation de polymères et composés spécifiques dans ces compositions de bain (thermo)gélifiantes et relaxantes, de préférence moussantes.

Les compositions selon l'invention sont essentiellement des compositions pour une application topique et notamment cosmétique ou dermatologique.

Le domaine technique de l'invention peut être défini de manière générale comme celui des bains gélifiants et moussants.

Il existe en effet actuellement des produits à mettre dans le bain, qui sont à la fois gélifiants et moussants, comme par exemple le produit japonais commercialisé sous la dénomination de ESTHE JELLY®. Les bains ainsi épaissis procurent une sensation d'apensateur extrêmement relaxante.

De telles compositions sont décrites, par exemple, dans les documents suivants.

Le document JP-A-61(86)-68411 décrit un procédé pour appliquer et absorber une substance active sur un corps humain, dans lequel une poudre d'une résine ayant une grande capacité d'absorption d'eau est ajoutée à de l'eau ou à de l'eau chaude dans une baignoire, moyennant quoi l'eau est transformée en un agrégat aqueux fluide de particules. Un ingrédient actif est ensuite ajouté, qui est maintenu uniformément dans les espaces entre les granules ou particules de l'agrégat de particules. La substance active peut être un cosmétique huileux, un agent nutritif, une vitamine, ou un composé pharmaceutique.

La résine qui a une grande capacité d'absorption d'eau est, de préférence, un composé polymère de masse moléculaire élevée contenant un grand nombre de groupes carboxyle et hydroxyle. Lorsqu'une poudre d'une telle résine est placée dans l'eau, ses granules absorbent l'eau, gonflent et prennent la forme de particules aqueuses sphériques, qui même lorsqu'elles sont au contact les unes des autres ne coagulent pas, mais glissent plutôt librement les unes sur les autres pour donner un agrégat de particules, aqueux, fluide.

Un polymère préféré est l'IGETAGEL® P qui serait un copolymère d'alcool vinylique, d'acétate de vinyle, d'acide acrylique et d'acrylate de méthyle, dans lequel les groupes carboxyle de l'acide acrylique ont été transformés en leur sels de sodium. Une telle résine pulvérulente a une capacité d'absorption de l'eau de 400 à 500 fois son propre poids.

Le document JP-A-62(87)-106669 décrit un produit pour le bain, comprenant un composé de masse moléculaire élevée à grande capacité d'absorption d'eau, en tant qu'ingrédient essentiel. Le composé est choisi parmi les résines préparées par polymérisation d'un produit de l'acide polyacrylique neutralisé par la soude, un produit réticulé tridimensionnellement de l'acrylate de sodium, un composé préparé par polymérisation par greffage de l'acrylonitrile sur l'amidon, et un copolymère acide acrylique - alcool vinylique.

Ces composés absorbent l'eau à raison de 300 à 1 000 fois leur propre poids et solidifient sous la forme d'un gel. Lorsqu'ils sont dissous dans l'eau ou l'eau chaude, par exemple d'une baignoire, en une quantité de 1 000 à 2 000 fois leur propre poids, ils prennent alors la forme d'une gelée visqueuse semi-fluide.

Le document JP-A-6(94)-172155 est relatif à un produit pour le bain contenant au moins un épaississant choisi parmi les polysaccharides et leurs dérivés, les polymères synthétiques solubles dans l'eau et les matériaux argileux susceptibles de se dilater dans l'eau. La viscosité de l'eau dans laquelle cet épaississant est dissout dans une proportion de 0,5 % pds/vol. est de 10 cps ou plus.

Les polymères synthétiques sont choisis parmi les polymères carboxyvinyliques et leurs sels, l'alcool polyvinylique, la polyvinylpyrrolidone, le poly(méthacrylate de méthyle), l'acide polyacrylique neutralisé par la soude, le polyacrylamide et le poly(oxyde d'éthylène).

Utilisé dans un bain, le produit de ce document permet de maintenir sa température et celle du corps après le bain, et d'obtenir une température uniforme dans tout le bain.

La limite supérieure de la viscosité ne doit pas dépasser 1 000 000 cps, car au-delà les problèmes liés au nettoyage de la baignoire deviennent insurmontables.

Il ressort de ce qui précède que les compositions pour bain gélifié relaxant existantes présentent l'inconvénient majeur d'être difficiles à éliminer après le bain, en raison notamment de leur viscosité importante, aussi bien à la température élevée du bain qu'à plus faible température, suite au refroidissement de ce dernier. Une solution qui a été proposée pour remédier à ce problème consiste à ajouter du sel, afin de fluidifier le bain, mais les quantités de sel nécessaires sont importantes, ce qui rend cette solution peu pratique à mettre en oeuvre.

Il existe donc un besoin pour une composition de bain, c'est-à-dire pour une composition à mettre dans un bain qui, d'une part, gélifie dans les conditions d'utilisation, à savoir généralement à une température de l'eau de 30 à 50°C, et qui, d'autre part, permette une évacuation ou une vidange facile, par exemple de la baignoire, à l'issue du bain, cette évacuation se produisant généralement après refroidissement du bain.

Une telle composition doit, en outre, de préférence, être peu agressive pour la peau et former une mousse stable.

Le but de la présente invention est, entre autres, de répondre à ces besoins.

Le but de la présente invention est encore de fournir une composition de bain thermogélifiante ayant des propriétés relaxantes, qui ne présente pas les inconvénients, limitations, défauts et désavantages des compositions de l'art antérieur, et qui résolve les problèmes des compositions de l'art antérieur.

Ce but et d'autres encore sont atteints, conformément à l'invention, par une composition de bain gélifiante, comprenant au moins un polymère ou composé gélifiant, ledit polymère ou composé gélifiant étant un polymère ou composé thermogélifiant dont les propriétés de gélification par chauffage sont réversibles avec la température.

Selon un mode préféré de réalisation de l'invention, la composition est une composition moussante.

En outre, elle peut contenir un ou plusieurs tensioactifs, de préférence moussants.

Le polymère ou composé inclus dans la composition de bain selon l'invention apporte, d'une part, à cette composition, son état gélifié dans les conditions d'utilisation, c'est-à-dire à la température généralement relativement élevée, correspondant, par exemple, à la température courante d'un bain et, d'autre part, permet l'élimination aisée de la composition après le bain par simple refroidissement.

Plus précisément, au-delà d'une certaine température, généralement appelée température de gélification, le polymère ou composé, et de ce fait la composition, se présente sous la forme d'un gel, et en dessous de cette température, le polymère ou composé, et donc la composition, est d'une viscosité nettement inférieure, et la composition est alors généralement liquide et donc facile à évacuer.

Tous les composés ou polymères thermogélifiants présentant des propriétés de gélification par chauffage, réversibles avec la température conviennent pour une utilisation dans la composition de l'invention.

Ces composés ou polymères peuvent être notamment choisis parmi les composés ou polymères suivants :
- les polymères comprenant des unités hydrosolubles et des unités présentant dans l'eau une température inférieure critique de démixtion (LCST) ;
- les dérivés cellulosiques, tels que la méthylcellulose et l'hydroxypropylméthylcellulose ;
- les copolymères à blocs de type polyoxyalkylénes ;
- les éthers de cellulose non ioniques ;
- les polymères hydrosolubles associatifs en présence d'au moins un agent tensioactif formant des structures en bicouches en solution aqueuse.

Parmi ces composés ou polymères, les polymères comprenant des unités hydrosolubles et des unités présentant dans l'eau une température inférieure critique de démixtion LCST sont préférés car ils permettent d'obtenir à de faibles concentrations des compositions aqueuses thermogélifiantes transparentes, moins sensibles à la présence de tensioactifs que les autres composés ou polymères.

La température de démixtion par chauffage en solution aqueuse desdites unités à LCST de ces derniers polymères est, de préférence, de 5 à 40°C pour une concentration massique dans l'eau de 1 % desdites unités.

De manière surprenante, il a été montré que les compositions de bain, gélifiantes selon l'invention, qui comprennent le polymère ou composé spécifique, tel qu'il est défini ci-dessus, permettent de satisfaire aux besoins énumérés ci-dessus et de répondre à l'ensemble des exigences mentionnées dans ce qui précède.

Notamment, le polymère ou composé permet d'obtenir une composition de bain qui gélifie le bain dans les conditions courantes d'utilisation, à savoir à une température de l'eau allant généralement de 30 à 50°C, mais qui, de manière surprenante, permet, à l'issue du bain, une évacuation, une vidange aisées du récipient, tel qu'une baignoire, dans lequel se trouve le bain, par simple refroidissement.

En d'autres termes, à l'issue du bain, c'est-à-dire lorsqu'il s'est produit un refroidissement du contenu du récipient dans lequel celui-ci a eu lieu, l'évacuation ou la vidange dudit récipient est aisée car la viscosité du bain, à cause de la présence du polymère ou composé spécifique selon l'invention, est réduite à ces températures plus froides, par exemple de 5 à 30°C. A titre d'exemple, la viscosité est alors de 0,001 à 0,5 Pa.s, ce qui correspond à un état fluide ou quasi fluide d'écoulement aisé.

Par évacuation ou vidange, facile, aisée, on entend généralement que le contenu du récipient, tel que la baignoire, est fluide, peu visqueux et peut s'écouler sans intervention extérieure et sous l'effet de la seule gravité dans le ou les orifices d'évacuation, de vidange, normalement prévus à cet effet.

Au contraire des compositions de l'art antérieur, du fait des propriétés du polymère ou composé spécifique inclus dans les compositions de l'invention, il se produit une diminution de la viscosité du contenu du récipient de bain lors du refroidissement de ce dernier, alors que, avec les compositions de l'art antérieur, la viscosité du bain reste constante et élevée, quelle que soit la température, ce qui rend très difficile, voire même impossible l'évacuation ou la vidange du récipient où à lieu le bain.

De plus, pour permettre une évacuation ou vidange facile, contrairement aux compositions de l'art antérieur, il n'est pas nécessaire que la composition selon l'invention contienne du sel et notamment des quantités importantes de sels, puisque la diminution de la viscosité est due de manière inhérente aux propriétés du composé polymère.

En outre, les compositions selon l'invention peuvent mousser tout en ne comportant généralement que peu ou pas de tensioactifs.

De ce fait, les compositions ou formules de bain selon l'invention présentent une très bonne innocuité vis-à-vis de la peau.

Les compositions de bain (thermo)gélifiantes, selon l'invention ont en général des propriétés moussantes importantes et produisent une mousse stable, notamment au-delà de 30°C et notamment en présence d'eau chaude à de 30 à 50°C.

Il est à noter que par composition de bain, selon l'invention, on entend une composition à mettre dans le bain, une composition à ajouter à un bain, c'est-à-dire à une quantité d'eau contenue dans un récipient, tel qu'une baignoire. L'addition de la composition selon l'invention à l'eau se trouvant dans ledit récipient qui est généralement à une température supérieure à la température de gélification du polymère ou composé, c'est-à-dire, par exemple, de 30 à 50°C, va former une structure de type gel-mousse dans ledit récipient. La consistance d'un tel gel-mousse et très relaxante.

De ce fait, la quantité de composition de bain selon l'invention à ajouter au contenu d'un récipient pour le bain est telle que soit obtenue une augmentation de la viscosité de ce contenu - c'est-à-dire essentiellement de l'eau - de façon à atteindre la consistance adéquate qui peut par exemple correspondre à une viscosité supérieure ou égale à 0,5 Pa.s, et par exemple de 0,5 à 100 Pa.s.

Lors du refroidissement à l'issue du bain, la température devient inférieure à la température de gélification (Tgel) du polymère ou composé et le bain a, du fait des propriétés de thermogélification réversibles des polymères ou composés mis en oeuvre selon l'invention, une viscosité comparativement très réduite, qui lui donne une consistance fluide.

L'incorporation de composés ou polymères à propriétés de thermogélification réversibles dans des compositions de bain n'est ni décrite ni suggérée dans l'art antérieur.

Les compositions de bain (thermo)gélifiantes selon l'invention peuvent se présenter sous toute forme à une température ambiante lors de leur stockage, avant d'être introduites dans la masse d'eau chaude formant le bain qui se trouve à une température qui est généralement supérieure à leur température de gélification.

Cela signifie que « dans le pot », c'est-à-dire avant leur introduction dans l'eau du bain, et préalablement à l'augmentation de température intervenant généralement à cette occasion, et préalablement à la formation éventuelle de mousse intervenant de même généralement au moment de cette utilisation, et liée généralement à l'agitation de l'eau du bain, la gamme des textures accessibles par les compositions de l'invention n'est pas limitée.

Les compositions de bain selon l'invention peuvent se présenter sous la forme de compositions solides, de préférence en particules ou de poudre, ou bien sous la forme de compositions liquides, éventuellement gélifiées, par exemple de solutions aqueuses concentrées.

Selon l'invention, la composition est, de préférence, une composition solide, sous la forme de particules : la taille et la forme de telles particules n'est pas limitée.

Il peut s'agir, par exemple, de billes, granulés, d'une poudre, ou autres.

On peut par exemple avoir des particules ayant une granulométrie allant de 1 µm à 5.10³ µm.

On peut faire varier à volonté la forme de la composition de l'invention, et cependant, quelle que soit cette forme, on aura lors de l'utilisation, c'est-à-dire de l'incorporation dans l'eau formant le bain, qui s'accompagne d'une augmentation de température (par exemple, à de 30 à 50°C), une apparition du pouvoir gélifiant du polymère ou composé et gélification avec formation éventuelle d'une mousse stable et durable. Cette texture disparaît lors du refroidissement du bain et celui-ci devient d'une faible viscosité et peut être facilement évacué, vidé.

De plus, il a été constaté que les polymères ou composés inclus dans la composition de l'invention, en particulier ceux comprenant des unités hydrosolubles et des unités à LCST, ne « tuaient pas » la mousse et que celle-ci était stable sur une longue durée.

L'invention a trait, en outre, à l'utilisation des polymères ou composés thermogélifiants dont les propriétés de gélification par chauffage sont réversibles avec la température, tels que décrits dans la présente description, pour gélifier un volume ou une masse d'eau à une température déterminée dans un récipient, et fluidifier ledit volume d'eau lors de son refroidissement, afin de faciliter l'évacuation dudit récipient.

Ladite température déterminée est généralement de 30 à 50°C, qui correspond couramment à celle d'un bain, tandis que la température après refroidissement est généralement de 5 à 30°C.

Le constituant essentiel des compositions selon l'invention est un composé ou polymère thermogélifiant dont les propriétés gélifiantes sont réversibles avec la température.

Ces polymères ou composés sont choisis notamment parmi les composés suivants :
- les polymères hydrosolubles constitués par des unités hydrosolubles et des unités présentant dans l'eau une température inférieure critique de démixtion (LCST) ;
- les dérivés cellulosiques, tels que la méthylcellulose et l'hydroxypropylméthylcellulose qui, en solution aqueuse à certaines concentrations (parexemple, voisines de 3 %), présentent des propriétés de thermogélification dans un domaine étroit de température, en dessous de leur point critique inférieur de démixtion (ou LCST). De tels dérivés sont décrits, par exemple, dans le document [1] ;
- les copolymères à blocs de type polyoxyalkylène (par exemple de 2 à 6 C), tel que les PLURONICS® (POE/POP/POE), qui présentent à des concentrations de l'ordre de 20 à 30 % en solution aqueuse, une transition sol/gel par élévation de la température, comme cela est mentionné dans le document [2]. Cette gélification par chauffage est réversible avec la température ;
- les éthers de cellulose non ioniques, tels que l'éthylhydroxyéthyl cellulose, qui en solution aqueuse montrent en présence de tensioactifs ioniques (tels que le sodium dodécyl sulfate) un pouvoir épaississant lorsque la température augmente, comme cela est décrit dans le document [3] ;
- les polymères hydrosolubles associatifs, en présence d'au moins un agent tensioactif formant des structures en bicouches en solution aqueuse, décrits dans le document [4]. Par exemple, un acide polyacrylique de masse molaire 150 000 g/mole, modifié par 3 % (en mole) de chaîne octadécyle présente en solution aqueuse à 1 % (en poids), en présence du tensioactif C₁₂(OE)₄ à 0,1 mole/l, des propriétés de gélification par élévation de la température.

Parmi les systèmes thermogélifiants réversibles, les polymères hydrosolubles constitués par des unités hydrosolubles et des unités présentant dans l'eau une température inférieure critique de démixtion (LCST) sont préférés car ils permettent d'obtenir à des faibles concentrations (quelques pour cents) des compositions aqueuses thermogélifiantes transparentes, moins sensibles à la présence de tensioactifs que les autres polymères ou composés décrits.

A ce propos, il est utile de rappeler que par unités à LCST, on entend des unités dont la solubilité dans l'eau est modifiée au-delà d'une certaine température. Il s'agit d'unités présentant une température de démixtion par chauffage (ou point de trouble) définissant leur zone de solubilité dans l'eau. La température de démixtion minimale obtenue en fonction de la concentration en polymère constitué uniquement d'unités à LCST est appelée « LCST » (Lower Critical Solution Température) . Pour chaque concentration en polymère à LCST, une température de démixtion par chauffage est observée. Elle est supérieure à la LCST qui est le point minimum de la courbe. En dessous de cette température, le polymère est soluble dans l'eau, au-dessus de cette température, le polymère perd sa solubilité dans l'eau.

Les unités à LCST du polymère préféré utilisé dans la composition selon l'invention répondent, de préférence, à une définition spécifique (proportions des unités LCST dans le polymère de 5 à 70 % de préférence de 20 à 65 %, et température de gélifification du polymère de 5 à 50°C pour une concentration massique de 2 % en poids dans l'eau), qui, de manière fondamentale, permet de communiquer au polymère les comprenant les propriétés de thermogélification avantageuses, décrites plus haut.

Les unités à LCST du polymère préféré ont, selon l'invention, une température de démixtion par chauffage de 5 à 50°C pour une concentration massique dans l'eau de 1 % en poids desdites unités à LCST.

De préférence, la température de démixtion par chauffage en solution aqueuse des unités à LCST du polymère préféré est de 15 à 40°C, et de préférence encore de 20 à 35°C, pour une concentration massique dans l'eau de 1 % desdites unités à LCST.

De préférence, la concentration du polymère ou composé, notamment du polymère préféré dans le bain final préparé à partir de la « composition de bain », c'est-à-dire de la composition à mettre dans le bain selon l'invention, est telle que la température de gélification du bain soit dans la plage de 20 à 35°C.

La concentration du polymère ou composé et notamment du polymère préféré dans le bain final sera généralement de 1 à 200 g/l pour obtenir la texture voulue à la température habituelle d'un bain qui est généralement de 30 à 50°C.

Le polymère préféré, inclus dans la composition de bain, ayant la structure décrite plus haut avec des unités hydrosolubles et des unités à LCST spécifiques définies ci-dessus présente en solution aqueuse, des propriétés de gélification au-delà d'une température critique, ou propriétés de thermogélification.

Ces propriétés de gélification par chauffage observées au-delà de la température de démixtion des chaînes à LCST sont décrites dans l'art antérieur, notamment dans les documents [5], [6] et [7]. Elles sont dues à l'association des chaînes à LCST au sein de microdomaines hydrophobes au-delà de leur température de démixtion, formant ainsi des noeuds de réticulation entre les chaînes principales.

Ces propriétés gélifiantes sont observées lorsque la concentration en polymère préféré est suffisante pour permettre des interactions entre des greffons à LCST portées par des macromolécules différentes. La concentration minimale nécessaire, appelée « concentration critique d'agrégation ou CAC », est évaluée par des mesures de rhéologie : il s'agit de la concentration à partir de laquelle la viscosité d'une solution aqueuse des polymères préférés inclus dans les compositions de l'invention devient supérieure à la viscosité d'une solution du polymère équivalent ne comportant pas de chaînes à LCST.

Au-delà de la CAC, les polymères préférés de l'invention présente des propriétés de gélification lorsque la température devient supérieure à une valeur critique appelée « température de gélification ou Tgel ». D'après les données de la littérature, un bon accord existe entre Tgel et la température de démixtion des chaînes à LCST, dans les mêmes conditions de concentrations. La température de gélification d'une solution aqueuse d'un polymère préféré inclus dans la composition de l'invention est déterminée par des mesures de rhéologie : il s'agit de la température à partir de laquelle la viscosité de la solution d'un polymère préféré inclus dans la composition de l'invention devient supérieure à la viscosité d'une solution du polymère équivalent ne comportant pas de chaînes à LCST.

Les polymères préférés de l'invention se caractérisent par une température de gélification spécifique généralement de 5 à 50°C, de préférence de 15 à 40°C, pour une concentration massique dans l'eau, par exemple égale à 2 % en poids.

Les autres polymères et composés inclus dans les compositions de l'invention ont des températures de gélification de 15 à 50°C pour une concentration massique dans l'eau, par exemple de 0,1 à 50 % en poids.

Cette température de gélification spécifique permet à ces polymères et composés, en particulier, aux polymères préférés, de communiquer aux compositions de l'invention toutes les propriétés avantageuses citées plus haut et, en particulier, leur texture mousse gélifiée à la température « chaude » du bain et la fluidité lors du refroidissement.

Des polymères ou composés thermogélifiants dont les propriétés gélifiantes sont réversibles avec la température sont connus de l'art antérieur, mais leur mise en oeuvre dans des bains, afin notamment d'en faciliter l'évacuation, n'est jamais décrite, ni suggérée.

En particulier, des polymères comportant, à l'instar de ceux mis en oeuvre, de préférence, dans les compositions de l'invention, des unités hydrosolubles et des unités à LCST et possédant des propriétés de gélification par chauffage observées au-delà de la température de démixtion des chaînes à LCST sont également décrits dans les documents déjà cités plus haut ([5], [6], [7]).

Le document [5] est relatif au thermo-épaississement réversible de solutions aqueuses de copolymère comprenant un squelette hydrosoluble d'acide polyacrylique avec des greffons de poly(oxyde d'éthylène) (PEO).

Le document [6] concerne le comportement thermo-épaississant en solution aqueuse de polymères comportant un squelette d'acide 2-acrylamido-2-méthylpropanesulfonique (AMPS) et des chaînes latérales de poly(oxyde éthylène).

De même, le document [7] décrit la thermo-association réversible de copolymères à squelette hydrosoluble polyacrylique ou à base d'AMPS avec des greffons de POE.

Les polymères, tels que ceux mentionnés dans les documents [5], [6] et [7] trouvent, en particulier, leur application dans l'industrie pétrolière.

Ainsi, le document [8] décrit-il des polymères thermoviscosifiants à squelette hydrosoluble comportant des segments, ou portant des chaînes latérales, à LCST qui peuvent être utilisés notamment en tant qu'agents épaississants, constituants de fluides de forage ou autres fluides, et fluides de nettoyage industriel.

Le document [9] décrit des polymères analogues à ceux du document [8] et leur utilisation en tant qu'agent anti-sédimentation de suspensions, éventuellement dans les préparations cosmétiques.

Il est à noter qu'aucun des documents [5] à [9] ne mentionne l'incorporation des polymères dans des compositions de bains gélifiantes et que, en outre, les polymères décrits ne comprennent pas les unités à LCST, spécifiques des polymères préférés selon l'invention.

Le document [10] décrit également les copolymères comprenant un squelette constitué par des unités sensibles au pH, par exemple des unités polyacryliques, et des unités sensibles à la température, greffées sur ce squelette. Ces copolymères présentent des propriétés de gélification en température et ils sont utilisés pour la libération et le relargage contrôlé de principes actifs ou pharmaceutiques et, éventuellement cosmétiques, par application topique. L'utilisation de ces polymères dans des produits pour le bain n'est pas citée.

Les unités sensibles à la température des copolymères de ce document ne sont pas les unités à LCST spécifiques des polymères préférés de l'invention. De plus, les polymères selon le document [10] se caractérisent par l'opacité extrêmement gênante des produits obtenus en température, ce qui n'est pas le cas des polymères préférés mis en oeuvre dans les compositions de l'invention.

En fait, le polymère de ce document est fondamentalement différent du polymère préféré de l'invention car il présente globalement pour l'ensemble du polymère une LCST dans la plage de températures de 20 à 40°C, contrairement aux polymères de l'invention qui sont hydrosolubles à toute température.

Les documents [11] et [12] décrivent des systèmes polymères à gélification réversible, comprenant un composant sensible capable d'agrégation, en réponse à un changement dans un « stimulus » extérieur, et un composant structural. Le stimulus extérieur peut être, par exemple, la température. Ainsi, il est indiqué que l'on obtient une augmentation de la viscosité d'au moins un facteur 30 pour une solution aqueuse de polymère à une concentration massique inférieure à 4 %.

Le composant sensible au stimulus extérieur est fondamentalement différent des unités à LCST des polymères préférés de la demande. En effet, ces composants sensibles au stimulus extérieur sont en fait constitués par au moins un fragment hydrophile et un fragment hydrophobe. Ainsi, le composant sensible peut être un copolymère bloc, tel qu'un « poloxamer », par exemple un PLURONIC®, qui est un polymère bloc d'oxyde éthylène (soluble) et d'oxyde de propylène (insoluble), un tel copolymère bloc s'agrège au niveau microscopique au-delà d'une température critique ne correspondant pas à une LCST. On peut aussi utiliser comme composant sensible un tensioactif non ionique.

Le document [11] concerne plus particulièrement un réseau polymérique formé d'un squelette hydrosoluble polyacrylique et d'un composant sensible de PLURONIC®, qui est enchevêtré dans ledit squelette, sans liaison covalente, ce réseau a donc une structure particulière qui n'a rien de commun avec le polymère préféré de l'invention. Par contre, dans le document [12], il est question de polymères avec des liaisons covalentes.

Ces polymères ont des propriétés de gélification par chauffage et ils peuvent être utilisés dans le domaine pharmaceutique pour la délivrance de médicaments et dans de nombreux autres domaines y compris le domaine des cosmétiques. En ce qui concerne les applications cosmétiques citées seuls des exemples d'émulsions sont mentionnés. Elles contiennent toutes un tensioactif neutre, anionique, ou cationique, ainsi que le polymère jouant le rôle de gélifiant.

L'utilisation des polymères dans des bains gélifiants n'est jamais évoquée, ni notamment les effets de vidange facile par refroidissement obtenus dans l'invention.

Dans ces formulations, le composant sensible du système polymère a un comportement différent de celui d'unités à LCST, telles que celles du polymère préféré de l'invention, lors du chauffage. Ainsi, lorsqu'on chauffe ledit composant sensible (par exemple, poloxamer), à environ 30-40°C, il présente une température de micellisation, c'est-à-dire une agrégation à l'échelle microscopique, puis lorsqu'on le chauffe davantage, une température de LCST très supérieure. Cette LCST correspond à une agrégation à l'échelle macroscopique entre les macromolécules. Il est expliqué dans WO-A-97 00275 [12] aux pages 16 et 17, que la gélification et la LCST sont observées à des températures qui diffèrent d'environ 70°C, la température de gélification correspondant à la température de micellisation du composant sensible, ce qui montre que ces polymères sont différents de ceux, préférés, de notre demande. En outre, il n'est pas possible, du fait de la synthèse utilisée dans le document [12], de contrôler parfaitement la structure et les propriétés du polymère final obtenu, comme c'est le cas dans les compositions de l'invention, en particulier pour les polymères préférés.

On connaît encore par le document [13] des compositions cosmétiques utilisant un système polymère à thermogélification réversible, comprenant l'acide polyacrylique et un poloxamer comme dans les documents [11] et [12]. De nouveau, le système polymère de ces documents est fondamentalement différent de ceux mis en oeuvre dans les compositions de l'invention, si bien que les propriétés avantageuses des compositions de l'invention ne peuvent être obtenues.

WO-A-00 35961 [14] décrit la préparation de polymères ayant des propriétés d'épaississement en température par polymérisation en émulsion et l'utilisation de ces polymères dans des compositions pharmaceutiques et cosmétiques. Ces polymères peuvent être des copolymères ayant des unités hydrosolubles et des unités à LCST à base d'oxyde d'alkylène. Il est prévu d'ajouter des tensioactifs non ioniques aux polymères pour renforcer leurs propriétés thermo-épaississantes.

Il ressort de ce qui précède que la mise en oeuvre dans des compositions de bain gélifiantes, moussantes, des polymères ou composés selon l'invention - et en particulier la mise en oeuvre des polymères préférés selon l'invention, présentant des unités à LCST spécifiques - conférant à ces bains des propriétés de gélification à la température d'utilisation allant de pair avec une viscosité réduite sous l'effet d'un refroidissement, n'est ni décrite ni suggérée dans les documents de l'art antérieur.

Les polymères préférés (à unités hydrosolubles et à unités à LCST) utilisés dans l'invention peuvent être des polymères séquencés (ou blocs), ou des polymères greffés, qui comprennent, d'une part, des unités hydrosolubles et, d'autre part, des unités à LCST telles que définies ci-dessus.

On précise que, dans le présent texte, les unités hydrosolubles ou les unités à LCST des polymères préférés mis en oeuvre selon l'invention sont définies comme n'incluant pas les groupes liant entre elles, d'une part, lesdites unités hydrosolubles et, d'autre part, lesdites unités à LCST.

Lesdits groupes de liaison sont issus de la réaction, lors de la préparation du polymère préféré, des sites réactifs portés, d'une part, par les précurseurs desdites unités hydrosolubles et, d'autre part, par les précurseurs desdites unités à LCST.

Les polymères préférés employés dans le cadre de l'invention peuvent donc être des polymères séquencés, comprenant par exemple des séquences constituées d'unités hydrosolubles alternées avec des séquences à LCST.

Ces polymères peuvent également se présenter sous la forme de polymères greffés dont le squelette est formé d'unités hydrosolubles, ledit squelette étant porteur de greffons constitués d'unités à LCST.

Lesdits polymères peuvent être partiellement réticulés.

Par unités hydrosolubles, on entend généralement que ces unités sont des unités solubles dans l'eau, à une température de 5 à 80°C, à raison d'au moins 10 g/l, de préférence d'au moins 20 g/l.

Toutefois, on entend également par unités hydrosolubles des unités ne possédant pas obligatoirement la solubilité ci-dessus mentionnée, mais qui en solution aqueuse à 1 % en poids, de 5 à 80°C, permettent l'obtention d'une solution macroscopiquement homogène et transparente, c'est-à-dire ayant une valeur de transmittance maximum de la lumière, quelle que soit la longueur d'onde comprise entre 400 et 800 nm, à travers un échantillon de 1 cm d'épaisseur, d'au moins 85 %, de préférence d'au moins 90 %.

Ces unités hydrosolubles ne présentent pas de température de démixtion par chauffage de type LCST.

Ces unités hydrosolubles sont en totalité ou en partie susceptibles d'être obtenues par polymérisation, notamment radicalaire, ou par polycondensation, ou encore sont constituées en totalité ou en partie par des polymères naturels ou naturels modifiés, existants.

A titre d'exemple, les unités hydrosolubles sont en totalité ou en partie susceptibles d'être obtenus par polymérisation, notamment radicalaire, d'au moins un monomère choisi parmi les monomères suivants :
1) l'acide (méth)acrylique ;
2) les monomères vinyliques de formule (I) suivante : 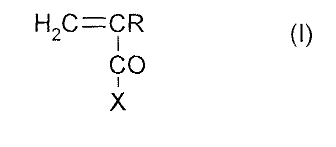 dans laquelle :
   - R est choisi parmi H, -CH₃, -C₂H₅ ou -C₃H₇ ; et
   - X est choisi parmi :
      - les oxydes d'alkyle de type -OR' où R' est un radical hydrocarboné, linéaire ou ramifié, saturé ou insaturé, ayant de 1 à 6 atomes de carbone, éventuellement substitué par au moins un atome d'halogène (iode, brome, chlore, fluor) ; un groupement sulfonique (-SO₃⁻), sulfate (-SO₄⁻), phosphate (-PO₄H₂) ; hydroxy (-OH) ; amine primaire (-NH₂) ; amine secondaire (-NHR₁), tertiaire (-NR₁R₂) ou quaternaire (-N⁺R₁R₂R₃) avec R₁, R₂ et R₃ étant, indépendamment l'un de l'autre, un radical hydrocarboné, linéaire ou ramifié, saturé ou insaturé ayant 1 à 6 atomes de carbone, sous réserve que la somme des atomes de carbone de R' + R₁ + R₂ + R₃ ne dépasse pas 7 ; et
      - les groupements -NH₂, -NHR₄ et -NR₄R₅ dans lesquels R₄ et R₅ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₄ + R₅ ne dépasse pas 7, lesdits R₄ et R₅ étant éventuellement substitués par un atome d'halogène (iode, brome, chlore, fluor) ; un groupement hydroxy (-OH) ; sulfonique (-SO₃⁻) ; sulfate (-SO₄⁻) ; phosphate (-PO₄H₂) ; amine primaire (-NH₂) ; amine secondaire (-NHR₁), tertiaire (-NR₁R₂) et/ou quaternaire (-N⁺R₁R₂R₃) avec R₁, R₂ et R₃ étant, indépendamment l'un de l'autre, un radical hydrocarboné, linéaire ou ramifié, saturé ou insaturé ayant 1 à 6 atomes de carbone, sous réserve que la somme des atomes de carbone de R₄ + R₅ + R₁ + R₂ + R₃ ne dépasse pas 7 ;
      - comme monomère vinylique de formule (I), on peut citer notamment l'acide 2-acrylamido-2-méthylpropane sulfonique (AMPS) - ou l'acide (méth)acrylique.
3) l'anhydride maléique ;
4) l'acide itaconique ;
5) l'alcool vinylique de formule CH₂=CHOH ;
6) l'acétate de vinyle de formule CH₂=CH-OCOCH₃ ;
7) les N-vinyllactames tels que la N-vinylpyrrolidone, la N-vinylcaprolactame et la N-butyrolactame ;
8) les vinyléthers de formule CH₂=CHOR₆ dans laquelle R₆ est un radical hydrocarboné, linéaire ou ramifié, saturé ou insaturé, ayant de 1 à 6 carbones ;
9) les dérivés hydrosolubles du styrène, notamment le styrène sulfonate ;
10) le chlorure de diméthyldiallyl ammonium ; et
11) la vinylacétamide.

Les polycondensats et les polymères naturels ou naturels modifiés qui peuvent constituer tout ou partie des unités hydrosolubles sont choisis parmi un ou plusieurs des composants suivants :
- les polyuréthanes hydrosolubles,
- la gomme de xanthane, notamment celle commercialisée sous les dénominations Keltrol T et Keltrol SF par Kelco ; ou Rhodigel SM et Rhodigel 200 de Rhodia ;
- les alginates (Kelcosol de Monsanto) et leurs dérivés tels que l'alginate de propylène glycol (Kelcoloid LVF de Kelco) ;
- les dérivés de cellulose et notamment la carboxyméthylcellulose (Aquasorb A500, Hercules), l'hydroxypropylcellulose, l'hydroxyéthylcellulose et l'hydroxyéthylcellulose quaternisée ;
- les galactomannanes et leurs dérivés, tels que la gomme Konjac, la gomme de guar, l'hydroxypropylguar l'hydroxypropylguar modifié par des groupements méthylcarboxylate de sodium (Jaguar XC97-1, Rhodia), le chlorure de guar hydroxypropyl triméthyl ammonium.

On peut également citer la polyéthylène imine.

De préférence, les unités hydrosolubles ont une masse molaire allant de 1 000 g/mole à 50 000 000 g/mole lorsqu'elles constituent le squelette hydrosoluble d'un polymère greffé.

Ces unités hydrosolubles ont de préférence une masse molaire allant de 500 g/mole à 100 000 g/mole lorsqu'elles constituent un bloc d'un polymère multiblocs.

De préférence, les unités hydrosolubles comportent au moins un monomère choisi parmi l'acide (méth)acrylique et l'acide 2-acrylamide-2-méthylpropane sulfonique.

On peut définir les unités à LCST des polymères, en particulier des polymères préférés utilisés dans l'invention, comme étant des unités dont la solubilité dans l'eau est modifiée au-delà d'une certaine température. Il s'agit d'unités présentant une température de démixtion par chauffage (ou point de trouble) définissant leur zone de solubilité dans l'eau. La température de démixtion minimale obtenue en fonction de la concentration en polymère est appelée "LCST" (Lower Critical Solution Température). Pour chaque concentration en polymère, une température de démixtion par chauffage est observée ; elle est supérieure à la LCST qui est le point minimum de la courbe. En dessous de cette température, le polymère constituant l'unité à LCST est soluble dans l'eau ; au-dessus de cette température, le polymère constituant l'unité à LCST perd sa solubilité dans l'eau.

Certains de ces polymères à LCST sont notamment décrits dans les articles de TAYLOR et al., Journal of Polymer Science, part A : Polymer Chemistry, 1975, 13, 2 551 [15] ; de J. BAILEY et al., Journal of Applied Polymer Science, 1959, 1,56 [16] ; et de HESKINS et al., Journal of Macromolecular Science, Chemistry A2, 1968, vol. 8, 1 441 [17].

Par soluble dans l'eau à la température T, on entend que les unités présentent une solubilité à T, d'au moins 1 g/l, de préférence d'au moins 2 g/l.

La mesure de la LCST peut se faire visuellement : on détermine la température à laquelle apparaît le point de trouble de la solution aqueuse ; ce point de trouble se traduit par l'opacification de la solution, ou perte de transparence.

D'une manière générale, une composition transparente aura une valeur de transmittance maximum de la lumière, quelle que soit la longueur d'onde comprise entre 400 et 800 nm, à travers un échantillon de 1 cm d'épaisseur, d'au moins 85 %, de préférence d'au moins 90 %.

La transmittance peut être mesurée en plaçant un échantillon de 1 cm d'épaisseur dans le rayon lumineux d'un spectrophotomètre travaillant dans les longueurs d'onde du spectre lumineux.

Les unités à LCST des polymères préférés utilisés dans l'invention peuvent être constituées par un ou plusieurs polymères choisis parmi les polymères suivants :
- les polyéthers tels que le poly oxyde d'éthylène (POE), le poly oxyde de propylène (POP), les copolymères statistiques d'oxyde d'éthylène (OE) et d'oxyde de propylène (OP),
- les polyvinylméthyléthers,
- les dérivés polymériques et copolymériques N-substitués de l'acrylamide ayant une LCST, tels que le poly N-isopropylacrylamide (Nipam) et le poly N-éthylacrylamide, et
- le poly-N-vinylcaprolactame et les copolymères de N-vinyl caprolactame.

De préférence, les unités à LCST sont constituées de poly oxyde de propylène (OP)ₙ où n est un nombre entier de 10 à 50, ou de copolymères statistiques d'oxyde d'éthylène (OE) et d'oxyde de propylène (OP), représentés par la formule :

(OE)ₘ (OP)ₙ

dans laquelle m est un nombre entier allant de 1 à 40, de préférence de 2 à 20, et n est un nombre entier allant de 10 à 60, de préférence de 20 à 50.

De préférence, la masse molaire de ces unités à LCST est de 500 à 5 300 g/mole, de préférence encore de 1 500 à 4 000 g/mole.

On a constaté que la répartition statistique des motifs OE et OP se traduit par l'existence d'une température inférieure critique de démixtion, au-delà de laquelle une séparation de phases macroscopique est observée. Ce comportement est différent de celui des copolymères (OE) (OP) à blocs, qui micellisent au-delà d'une température critique dite de micellisation (agrégation à l'échelle microscopique).

Les unités à LCST peuvent donc notamment être des copolymères statistiques d'oxyde d'éthylène et d'oxyde de propylène, aminés, notamment monoaminés, diaminés ou triaminés. Ces polymères, avant réaction, sont porteurs de sites réactifs, dans ce cas de groupes aminés, réagissant avec les sites réactifs des polymères hydrosolubles, par exemple des groupes carboxyle, pour donner le polymère final mis en oeuvre dans l'invention. Dans le polymère final, les unités hydrosolubles sont liés aux unités à LCST par des groupes de liaison issus de la réaction des groupes ou sites réactifs portés respectivement par les unités à LCST et les précurseurs des unités hydrosolubles. Ces groupes de liaison seront, par exemple, des groupes amide, ester, éthers ou uréthanes.

Parmi ces polymères à LCST, commercialement disponibles, on peut citer les copolymères vendus sous la dénomination Jeffamine par HUNTSMAN, et notamment la Jeffamine XTJ-507 (M-2005), la Jeffamine D-2000 et la Jeffamine XTJ-509 (ou T-3000).

Les unités à LCST peuvent également être issues de copolymères OE/OP statistiques à extrémités OH, tels que ceux vendus sous la dénomination Polyglycols P41 et B11 par Clariant.

On peut aussi utiliser dans les polymères préférés de l'invention, comme unités à LCST, les dérivés polymériques et copolymériques N-substitués de l'acrylamide ayant une LCST, ainsi que le poly-N-vinyl caprolactame et les copolymères de N-vinylcaprolactame.

A titre d'exemples de dérivés polymériques et copolymériques N-substitués de l'acrylamide ayant une LCST, on peut citer le poly N-isopropylacrylamide, le poly N-éthylacrylamide et les copolymères de N-isopropylacrylamide (ou de N-éthylacrylamide) et d'un monomère vinylique choisi parmi les monomères ayant la formule (I) donnée ci-dessus, l'anhydride maléique, l'acide itaconique, la vinylpyrrolidone, le styrène et ses dérivés, le chlorure de diméthyldiallyl ammonium, la vinylacétamide, l'alcool vinylique, l'acétate de vinyle, les vinyléthers et les dérivés de l'acétate de vinyle.

La masse molaire de ces polymères est de préférence de 1 000 g/mole à 500 000 g/mole, de préférence de 2 000 à 50 000 g/mole.

La synthèse de ces polymères peut être réalisée par polymérisation radicalaire à l'aide d'un couple d'amorceurs tel que le chlorhydrate d'aminoéthanethiol, en présence de persulfate de potassium, afin d'obtenir des oligomères précurseurs ayant une extrémité réactive aminée.

A titre d'exemples de copolymères de N-vinylcaprolactame, on peut citer les copolymères de N-vinylcaprolactame et d'un monomère vinylique ayant la formule (I) donnée ci-dessus, ou d'un monomère choisi parmi l'anhydride maléique, l'acide itaconique, la N-vinylpyrrolidone, le styrène et ses dérivés, le chlorure de diméthyldiallyl ammonium, la vinylacétamide, l'alcool vinylique, l'acétate de vinyle, les vinyléthers et les dérivés de l'acétate de vinyle.

La masse molaire de ces polymères ou copolymères de vinylcaprolactame est généralement de 1 000 g/mole à 500 000 g/mole, de préférence de 2 000 à 50 000 g/mol.

La synthèse de ces composés peut être réalisée par polymérisation radicalaire à l'aide d'un couple d'amorceurs tel que le chlorhydrate d'aminoéthanethiol, en présence de persulfate de potassium, afin d'obtenir des unités à LCST ayant une extrémité réactive aminée.

La proportion massique des unités à LCST dans le polymère final est de préférence de 5 % à 70 %, notamment de 20 % à 65 %, et particulièrement de 30 % à 60 % en poids, par rapport au polymère final.

On a vu plus haut que la température de démixtion par chauffage desdites unités à LCST du polymère préféré utilisé dans l'invention est de 5 à 50°C, de préférence de 15 à 40°C, pour une concentration massique dans l'eau, de 1 % en poids desdites unités à LCST.

Les polymères préférés employés dans le cadre de l'invention peuvent être aisément préparés par l'homme du métier sur la base de ses connaissances générales, en utilisant des procédés de greffage, de copolymérisation ou de réaction de couplage.

Lorsque le polymère final se présente sous la forme d'un polymère greffé, notamment présentant un squelette hydrosoluble avec des chaînes latérales ou greffons à LCST, il est possible de le préparer par greffage des unités à LCST ayant au moins une extrémité réactive ou site réactif, notamment aminé(e), sur un polymère hydrosoluble formant le squelette, portant au minimum 10 % (en mole) de groupes réactifs tels que des fonctions acides carboxyliques. Cette réaction peut s'effectuer en présence d'un carbodiimide tel que le dicyclohexylcarbodiimide ou le chlorhydrate de 1-(3-diméthylaminopropyl)-3-éthyl carbodiimide, dans un solvant tel que la N-méthylpyrrolidone ou l'eau.

Une autre possibilité pour préparer des polymères greffés consiste à copolymériser par exemple un macromonomère à LCST (chaîne à LCST précédemment décrite avec une extrémité vinylique) et un monomère vinylique hydrosoluble tel que l'acide acrylique, ou les monomères vinyliques ayant la formule (I), tel que l'acide 2-acrylamido-2-méthylpropane sulfonique (AMPS).

Lorsque le polymère final se présente sous la forme d'un polymère séquencé ou à blocs, il est possible de le préparer par couplage entre des unités hydrosolubles et des unités à LCST, ces unités ayant à chaque extrémité des sites réactifs complémentaires.

Dans le cas des procédés de greffage et des procédés de couplage, les sites réactifs des unités à LCST peuvent être des fonctions amines notamment monoamines, diamines ou triamines, et des fonctions OH. Dans ce cas, les sites réactifs des unités hydrosolubles peuvent être des fonctions acides carboxyliques. Les groupes liant les unités hydrosolubles et les unités à LCST seront donc, par exemple, des groupes amide ou des groupes ester.

Comme on l'a vu précédemment, les compositions gélifiantes de l'invention peuvent se trouver sous une forme allant de la forme liquide à la forme solide (forme liquide, crémeuse, pâteuse ou solide), par exemple en particules.

La composition, lorsqu'elle est liquide, peut être plus ou moins fluide. Elle comporte essentiellement une phase aqueuse continue. Cette phase aqueuse continue se présente généralement sous la forme d'une solution concentrée, de préférence une solution aqueuse concentrée en polymère ou composé.

Selon l'invention, la phase aqueuse comprend un polymère ou composé thermogélifiant dont les propriétés gélifiantes sont réversibles avec la température. De préférence, il s'agit d'un polymère hydrosoluble comprenant des unités hydrosolubles et des unités à LCST, tel que défini ci-dessus.

Généralement, la solution est une solution concentrée, c'est-à-dire que la concentration massique en polymère ou composé de la phase aqueuse est de 0,1 à 90 %, de préférence de 0,5 à 90 %.

De manière générale, cette concentration doit être suffisante pour obtenir la structure gélifiée voulue en ajoutant une quantité limitée, par exemple de 100 g, de la composition selon l'invention dans un bain d'un volume courant, par exemple de 200 l.

Lorsque la composition selon l'invention est une composition solide, par exemple particulaire, et notamment pulvérulente. La concentration massique du polymère ou composé se situe dans des plages analogues, à savoir de 0,5 à 1 g, de polymère ou composé par g de composition solide, par exemple de poudre.

La composition gélifiante, selon l'invention, grâce à la présence du polymère ou composé spécifique, décrit plus haut, peut constituer une composition moussante, même avec pas ou peu de tensioactifs, c'est-à-dire que la concentration de tensioactif est, par exemple, égale ou inférieure à 5% en masse, de préférence de 1 à 5 %.

Quand la composition contient un ou plusieurs tensioactifs, selon l'utilisation finale, elle peut contenir jusqu'à 20 % en poids de tensioactifs, par exemple de 0,05 à 20 % et mieux de 0,1 à 15 % en poids par rapport au poids total de la composition.

Les tensioactifs moussants utilisables dans la composition de l'invention peuvent être des tensioactifs non ioniques, anioniques, amphotères ou zwitterioniques et leurs mélanges ; les tensioactifs non-ioniques étant préférés.

Comme tensioactifs non ioniques, on peut citer par exemple les alkylpolyglycosides (APG), les esters de polyols et d'acides gras, les esters de polyéthylène glycols et d'acide gras, les dérivés d'alcools gras et de polyols (éthers), et les dérivés oxyalkylénés (oxyéthylénés et/ou oxypropylénés) de ces composés. On peut citer aussi les esters de maltose, les alcools gras polyglycérolés, les dérivés de glucamine comme l'éthyl-2 hexyl oxycarbonyl N-méthyl glucamine, et leurs mélanges.

Comme alkylpolyglycosides, on peut citer notamment les alkylpolyglucosides, et par exemple le décylglucoside (Alkyl-C9/C11-polyglycoside (1.4)) comme le produit commercialisé sous la dénomination MYDOL 10 par la société Kao Chemicals, les produits commercialisés sous la dénomination PLANTAREN 2000 UP et PLANTACARE 2000 UP par la société Henkel, et le produit commercialisé sous la dénomination ORAMIX NS 10 par la société Seppic ; le caprylyl/capryl glucoside comme le produit commercialisé sous la dénomination ORAMIX CG 110 par la Société Seppic ou sous la dénomination LUTENSOL GD 70 par la Société BASF ; le laurylglucoside comme les produits commercialisés sous les dénominations PLANTAREN 1200 N et PLANTACARE 1200 par la société Henkel ; et le coco-glucoside comme le produit commercialisé sous la dénomination PLANTACARE 818/UP par la société Henkel, et leurs mélanges.

Les dérivés de maltose sont par exemple ceux décrits dans le document EP-A-0 566 438 [18], tels que l'O-octanoyl-6'-D-maltose, ou encore le O-dodécanoyl-6'-D-maltose décrit dans le document FR-A-2 739 556 [19].

Parmi les alcools gras polyglycérolés, on peut citer le dodécanediol polyglycérolé (3,5 moles de glycérol), produit commercialisé sous la dénomination CHIMEXANE NF par la société Chimex.

Comme tensioactifs anioniques, on peut utiliser par exemple les carboxylates, les dérivés des aminoacides, les alkyl sulfates, les alkyl éther sulfates, les sulfonates, les iséthionates, les taurates, les sulfosuccinates, les alkylsulfoacétates, les phosphates et alkylphosphates, les polypeptides, les dérivés anioniques d'alkyl polyglycoside, les savons d'acides gras, et leurs mélanges.

Comme carboxylates, on peut citer par exemple les sels alcalins de N-acylaminoacides ; les amidoéthercarboxylates (AEC) comme le lauryl amidoéther carboxylate de sodium (3 OE) commercialisé sous la dénomination AKYPO FOAM 30 par la société Kao Chemicals ; les sels d'acides carboxyliques polyoxyéthylénés, comme le lauryl éther carboxylate de sodium (C12-14-16 65/25/10) oxyéthyléné (6 OE) commercialisé sous la dénomination AKYPO SOFT 45 NV par la société Kao Chemicals ; les acides gras d'huile d'olive polyoxyéthylénés et de carboxyméthyl, comme le produit commercialisé sous la dénomination OLIVEM 400 par la société Biologia E Tecnologia ; le tri-décyl éther carboxylate de sodium oxyéthyléné (6 OE) commercialisé sous la dénomination NIKKOL ECTD-6NEX par la société Nikkol ; le 2-(2-Hydroxyalkyloxy) acétate de sodium commercialisé sous la dénomination BEAULIGHT SHAA par la société SANYO.

Les dérivés des aminoacides peuvent être choisis par exemple parmi les sarcosinates et notamment les acylsarcosinates comme le lauroylsarcosinate de sodium commercialisé sous la dénomination SARKOSYL NL 97 par la société Ciba ou commercialisé sous la dénomination ORAMIX L 30 par la société Seppic, le myristoyl sarcosinate de sodium, commercialisé sous la dénomination NIKKOL SARCOSINATE MN par la société Nikkol, le palmitoyl sarcosinate de sodium, commercialisé sous la dénomination NIKKOL SARCOSINATE PN par la société Nikkol ; les alaninates comme le N-lauroyl-N-méthylamidopropionate de sodium commercialisé sous la dénomination SODIUM NIKKOL ALANINATE LN 30 par la société Nikkol ou commercialisé sous la dénomination ALANONE ALE par la société Kawaken, et le N-lauroyl N-méthylalanine triéthanolamine, commercialisé sous la dénomination ALANONE ALTA par la société Kawaken ; les N-acylglutamates comme le monococoylglutamate de triéthanolamine commercialisé sous la dénomination ACYLGLUTAMATE CT-12 par la société Ajinomoto, le lauroyl-glutamate de triéthanolamine commercialisé sous la dénomination ACYLGLUTAMATE LT-12 par la société Ajinomoto et le N-lauroyl-L-glutamate monosodique commercialisé sous la dénomination AMISOFT LS-11 par la société Ajinomoto ; les aspartates comme le mélange de N-lauroylaspartate de triéthanolamine et de N-myristoylaspartate de triéthanolamine, commercialisé sous la dénomination ASPARACK LM-TS2 par la société Mitsubishi ; les citrates, et leurs mélanges.

Comme dérivés de la glycine, on peut citer le N-cocoylglycinate de sodium et le N-cocoylglycinate de potassium comme les produits commercialisés sous les dénominations AMILITE GCS-12 et AMILITE GCK-12 par la société Ajinomoto.

Comme alkyl éther sulfates, on peut citer par exemple le lauryl éther sulfate de sodium (C12-14 70/30) (2,2 OE) commercialisé sous les dénominations SIPON AOS 225 ou TEXAPON N702 PATE par la société Henkel, le lauryl éther sulfate d'ammonium (C12-14 70/30) (3 OE) commercialisé sous la dénomination SIPON LEA 370 par la société Henkel, l'alkyl (C12-C14) éther (9 OE) sulfate d'ammonium commercialisé sous la dénomination RHODAPEX AB/20 par la société Rhodia Chimie.

Comme sulfonates, on peut citer par exemple les alpha-oléfines sulfonates comme l'alpha-oléfine sulfonate de sodium (C14-16) commercialisé sous la dénomination BIO-TERGE AS-40 par la société Stepan, commercialisé sous les dénominations WITCONATE AOS PROTEGE et SULFRAMINE AOS PH 12 par la société Witco ou commercialisé sous la dénomination BIO-TERGE AS-40 CG par la société Stepan, l'oléfine sulfonate de sodium secondaire commercialisé sous la dénomination HOSTAPUR SAS 30 par la société Clariant ; les alkyl aryl sulfonates linéaires comme le xylène sulfonate de sodium commercialisé sous les dénominations MANROSOL SXS30, MANROSOL SXS40, MANROSOL SXS93 par la société Manro.

Comme iséthionates, on peut citer les acyliséthionates comme le cocoyl-iséthionate de sodium, tel que le produit commercialisé sous la dénomination JORDAPON CI P par la société Jordan.

Comme taurates, on peut citer le sel de sodium de méthyltaurate d'huile de palmiste commercialisé sous la dénomination HOSTAPON CT PATE par la société Clariant ; les N-acyl N-méthyltaurates comme le N-cocoyl N-méthyltaurate de sodium commercialisé sous la dénomination HOSTAPON LT-SF par la société Clariant ou commercialisé sous la dénomination NIKKOL CMT-30-T par la société Nikkol, le palmitoyl méthyltaurate de sodium commercialisé sous la dénomination NIKKOL PMT par la société Nikkol.

Comme sulfosuccinates, on peut citer par exemple le monosulfosuccinate d'alcool laurylique (C12/C14 70/30) oxyéthyléné (3 OE) commercialisé sous les dénominations SETACIN 103 SPECIAL, REWOPOL SB-FA 30 K 4 par la société Witco, le sel di-sodique d'un hémisulfosuccinate des alcools C12-C14, commercialisé sous la dénomination SETACIN F SPECIAL PASTE par la société Zschimmer Schwarz, l'oléamidosulfosuccinate di-sodique oxyéthyléné (2 OE) commercialisé sous la dénomination STANDAPOL SH 135 par la société Henkel, le monosulfosuccinate d'amide laurique oxyéthyléné (5 OE) commercialisé sous la dénomination LEBON A-5000 par la société Sanyo, le sel di-sodique de monosulfosuccinate de lauryl citrate oxyéthyléné (10 OE) commercialisé sous la dénomination REWOPOL SB CS 50 par la société Witco, le monosulfosuccinate de monoéthanolamide ricinoléique commercialisé sous la dénomination REWODERM S 1333 par la société Witco.

Comme phosphates et alkylphosphates, on peut citer par exemple les monoalkylphosphates et les dialkylphosphates, tels que le monophosphate de lauryle commercialisé sous la dénomination MAP 20 par la société Kao Chemicals, le sel de potassium de l'acide dodécylphosphorique, mélange de mono- et di-ester (diester majoritaire) commercialisé sous la dénomination CRAFOL AP-31 par la société Cognis, le mélange de monoester et de di-ester d'acide octylphosphorique, commercialisé sous la dénomination CRAFOL AP-20 par la société Cognis, le mélange de monoester et de diester d'acide phophorique de 2-butyloctanol éthoxylé (7 moles d'OE), commercialisé sous la dénomination ISOFOL 12 7 EO-PHOSPHATE ESTER par la société Condea, le sel de potassium ou de triéthanolamine de monoalkyl (C12-C13) phosphate commercialisé sous les références ARLATONE MAP 230K-40 et ARLATONE MAP 230T-60 par la société Uniqema, le lauryl phosphate de potassium commercialisé sous la dénomination DERMALCARE MAP XC-99/09 par la société Rhodia Chimie.

Les polypeptides sont obtenus par exemple par condensation d'une chaîne grasse sur les aminoacides de céréale et notamment du blé et de l'avoine. Comme polypeptides, on peut citer par exemple le sel de potassium de la lauroyl protéine de blé hydrolysée, commercialisé sous la dénomination AMINOFOAM W OR par la société Croda, le sel de triéthanolamine de cocoyl protéine de soja hydrolysée, commercialisé sous la dénomination MAY-TEIN SY par la société Maybrook, le sel de sodium des lauroyl aminoacides d'avoine, commercialisé sous la dénomination PROTEOL OAT par la société Seppic, l'hydrolysat de collagène greffé sur l'acide gras de coprah, commercialisé sous la dénomination GELIDERM 3000 par la société Deutsche Gelatine, les protéines de soja acylées par des acides de coprah hydrogénés, commercialisées sous la dénomination PROTEOL VS 22 par la société Seppic.

Les dérivés anioniques d'alkylpolyglycosides peuvent être notamment des citrates, tartrates, sulfosuccinates, carbonates et éthers de glycérol obtenus à partir des alkyl polyglycosides et notamment de poly-alkyl-glucosides. On peut citer par exemple le sel de sodium d'ester tartrique de cocoylpolyglycoside (1,4), commercialisé sous la dénomination EUCAROL AGE-ET par la société Cesalpinia, le sel di-sodique d'ester sulfosuccinique de cocoylpolyglycoside (1,4), commercialisé sous la dénomination ESSAI 512 MP par la société Seppic, le sel de sodium d'ester citrique de cocoyl polyglycoside (1,4) commercialisé sous la dénomination EUCAROL AGE-EC® par la société Cesalpinia. Un autre dérivé d'holoside anionique peut être le dodécyl-D-galactoside uronate de sodium commercialisé sous la dénomination DODECYL-D-GALACTOSIDE URONATE DE SODIUM par la société SOLIANCE.

Les savons d'acide gras qui peuvent être utilisés comme tensioactifs anioniques sont des acides gras d'origine naturelle ou synthétique, salifiés par une base minérale ou organique. La chaîne grasse peut comporter de 6 à 22 atomes de carbone, de préférence de 8 à 18 atomes de carbone. La base minérale ou organique peut être choisie parmi les métaux alcalins ou alcalino-terreux, les aminoacides et les aminoalcools. Comme sels, on peut utiliser par exemple les sels de sodium, de potassium, de magnésium, de triéthanolamine, de N-méthylglucamine, de lysine et d'arginine. Comme savons, on peut citer par exemple les sels de potassium ou de sodium des acides laurique, myristique, palmitique, stéarique (laurate, myristate, palmitate et stéarate de potassium ou de sodium), et leurs mélanges.

Comme tensioactifs amphotères et zwitterioniques, on peut utiliser par exemple, les bétaïnes, les N-alkylamidobétaïnes et leurs dérivés, les dérivés de la glycine, les sultaïnes, les alkyl polyaminocarboxylates, les alkylamphoacétates et leurs mélanges.

Comme bétaïnes, on peut citer par exemple la cocobétaïne comme le produit commercialisé sous la dénomination DEHYTON AB-30 par la société Henkel, la laurylbétaïne comme le produit commercialisé sous la dénomination GENAGEN KB par la société Clariant, la laurylbétaïne oxyéthylénée (10 OE), comme le produit commercialisé sous la dénomination LAURYLETHER(10 OE)BETAINE par la société Shin Nihon Rica, la stéarylbétaïne oxyéthylénée (10 OE) comme le produit commercialisé sous la dénomination STEARYLETHER(10 OE)BETAINE par la société Shin Nihon Rica.

Parmi les N-alkylamidobétaines et leurs dérivés, on peut citer par exemple la cocamidopropyl bétaine commercialisée sous la dénomination LEBON 2000 HG par la société Sanyo, ou commercialisée sous la dénomination EMPIGEN BB par la société Albright & Wilson, la lauramidopropyl bétaïne commercialisée sous la dénomination REWOTERIC AMB12P par la société Witco.

Comme sultaïnes, on peut citer le cocoylamidopropylhydroxy-sulfobétaine commercialisé sous la dénomination CROSULTAINE C-50 par la société Croda.

Comme alkyl polyaminocarboxylates (APAC), on peut citer le cocoylpolyamino-carboxylate de sodium, commercialisé sous la dénomination AMPHOLAK 7 CX/C,et AMPHOLAK 7 CX par la société Akzo Nobel, le stéarylpolyamidocarboxylate de sodium commercialisé sous la dénomination AMPHOLAK 7 TX/C par la société Akzo Nobel, la carboxyméthyloléyl-polypropylamine de sodium, commercialisé sous la dénomination AMPHOLAK XO7/C par la société Akzo Nobel.

Comme alkylamphoacétates, on peut citer par exemple le N-cocoyl-N-carboxyméthoxyéthyl-N-carboxyméthyl-éthylènediamine N-di-sodique (nom CTFA : disodium cocamphodiacetate) comme le produit commercialisé sous la dénomination MIRANOL C2M CONCENTRE NP par la société Rhodia Chimie, et le N-cocoyl-N-hydroxyéthyl-N-carboxyméthyl-éthylènediamine N-sodique (nom CTFA : sodium cocamphoacétate).

Lorsque la composition selon l'invention est sous une forme solide, particulaire, de préférence pulvérulente, on préfère, parmi les tensioactifs, utiliser ceux pouvant se présenter sous forme de poudre qui sont, par exemple, le cocoylisethionate de sodium, le mono-sulfosuccinate de mono-éthanolamide ricinoléique, commercialisé sous la dénomination REWODERM S 1333 par la Société WITCO, le N-lauroyl-L-glutamate monosodique (AMISOFT LS-11) et le palmitoyl méthyltaurate de sodium, commercialisé sous la dénomination NIKKOL PMT par la Société NIKKOL.

La phase aqueuse peut éventuellement, en outre, comprendre un agent gélifiant à une concentration massique de 0,01 à 20 % du poids total de la composition, à la condition que le bain soit fluide à une température inférieure à 25°C.

Dans les compositions moussantes de l'invention, sous forme fluide, liquide, la phase aqueuse est de préférence constituée par un milieu physiologiquement acceptable permettant une application topique et notamment cosmétique.

On entend dans la présente demande par "milieu physiologiquement acceptable" un milieu compatible avec toutes les matières kératiniques telles que la peau y compris le cuir chevelu, les ongles, les muqueuses, les yeux et les cheveux ou toute autre zone cutanée du corps.

Le milieu physiologiquement acceptable des compositions moussantes de l'invention comprend de l'eau. La quantité d'eau peut aller de 30 à 99,98 % en poids et de préférence de 40 à 95 % en poids par rapport au poids total de la composition.

L'eau utilisée peut être outre de l'eau, une eau florale telle que l'eau de bleuet, une eau minérale telle que l'eau de Vittel, l'eau de LUCAS ou l'eau de la Roche Posay et/ou une eau thermale.

Le milieu physiologiquement acceptable peut contenir, outre l'eau, un ou plusieurs solvants choisis parmi les alcools inférieurs comportant de 1 à 8 atomes de carbone, tels que l'éthanol ; les polyols tels que la glycérine ; les glycols comme le butylène glycol, l'isoprène glycol, le propylène glycol, les polyéthylène glycols tels que le PEG-8 ; le sorbitol ; les sucres tels que le glucose, le fructose, le maltose, le lactose et le sucrose ; et leurs mélanges. La quantité de solvant(s) peut aller de 0,5 à 30 % en poids et de préférence de 5 à 20 % en poids par rapport au poids total de la composition.

Les compositions gélifiantes de l'invention peuvent encore contenir des adjuvants habituels dans les domaines cosmétique et dermatologique, tels que les charges minérales ou organiques, les actifs hydrophiles ou lipophiles, les conservateurs, les gélifiants, les plastifiants, les antioxydants, les parfums, les absorbeurs d'odeur, les anti-mottants (agents évitant l'agglomération de particules solides), les séquestrants (EDTA), les ajusteurs de pH acides ou basiques ou des tampons, et des matières colorantes (pigments ou colorants ou nacres). Les quantités de ces différents additifs sont celles classiquement utilisées dans les domaines considérés, et par exemple de 0,01 à 20 % du poids total de la composition. Bien entendu, l'homme de l'art veillera à choisir le ou les éventuels composés à ajouter aux compositions moussantes selon l'invention de manière telle que les propriétés avantageuses attachées intrinsèquement à ces compositions ne soient pas, ou substantiellement pas, altérées par l'addition envisagée.

Par charges, il faut comprendre des particules incolores ou blanches, minérales ou de synthèse, lamellaires ou non lamellaires, destinées à donner du corps ou de la rigidité à la composition et/ou de la douceur, de la matité et de l'uniformité au maquillage. Comme charges, on peut notamment citer le talc, le mica, la silice, le nitrure de bore, l'oxychlorure de bismuth, le kaolin, les poudres de Nylon telles que Nylon-12 (Orgasol commercialisé par la société Atochem), les poudres de polyéthylène, le Téflon (poudres de polymères de tétrafluoroéthylène), les poudres de polyuréthanne, les poudres de polystyrène, les poudres de polyester, l'amidon éventuellement modifié, des microsphères de copolymères, telles que celles commercialisées sous les dénominations Expancel par la société Nobel Industrie, les microéponges comme le Polytrap commercialisé par la société Dow Corning, les microbilles de résine de silicone telles que celles commercialisées par la société Toshiba sous la dénomination Tospearl, le carbonate de calcium précipité, le carbonate et l'hydrocarbonate de magnésium, l'hydroxyapatite, les microsphères de silice creuses (Silica Beads de la Société Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, et leurs mélanges.

Par pigments, il faut comprendre des particules blanches ou colorées, minérales ou organiques, insolubles dans le milieu, destinées à colorer et/ou opacifier la composition. Ils peuvent être blancs ou colorés, minéraux et/ou organiques, de taille usuelle ou nanométrique. On peut citer, parmi les pigments et les nanopigments minéraux, les dioxydes de titane, de zirconium ou de cérium, ainsi que les oxydes de zinc, de fer ou de chrome, les nanotitanes (nanopigments de dioxyde de titane), les nanozincs (nanopigments d'oxyde de zinc), le bleu ferrique. Parmi les pigments organiques, on peut citer le noir de carbone, et les laques comme les sels de calcium, de baryum, d'aluminium ou de zirconium, de colorants acides tels que les colorants halogéno-acides, azoïques ou anthraquinoniques.

Par nacres, il faut comprendre des particules irisées qui réfléchissent la lumière. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.

On peut ajouter aux compositions de l'invention, lorsqu'elles sont fluides, un agent gélifiant (différent du composé ou polymère gélifiant déjà décrit plus haut et dont les propriétés de gélification par chauffage sont réversibles avec la température) afin de régler la texture de la composition et d'accéder à une large gamme de textures allant du lait à la crème. Le composé gélifiant, dont il est question ici, est un composé gélifiant classique présentant des propriétés de gélification indépendantes de la température. Comme on l'a déjà mentionné plus haut, les compositions selon l'invention peuvent à faible température, par exemple à température ambiante, « dans le pot », revêtir toute texture souhaitable. Il n'y a aucune restriction sur la texture que la composition peut avoir, avant mise en oeuvre.

En particulier, la composition ne doit pas contenir obligatoirement un agent gélifiant autre que le composé ou polymère pour que lors de l'utilisation, on obtienne une mousse gélifiée. En effet, grâce au polymère ou composé spécifique inclus dans la composition de l'invention, la mousse obtenue lors de l'augmentation de température se produisant, par exemple, lors de l'utilisation de la composition, est gélifiée, stable, quelle que soit la texture, la forme de la composition de bain avant utilisation. On n'inclura donc un gélifiant dans la composition que si on souhaite que celle-ci ait un aspect gélifié, cet aspect n'étant selon l'invention qu'un aspect particulier parmi la multitude d'aspects, de textures, de formes que peut avoir la composition de bain.

Selon un mode préféré de réalisation de l'invention, la composition a pas ou peu d'agent gélifiant (moins de 0,1 %).

Les gélifiants utilisables peuvent être des gélifiants hydrophiles. A titre d'exemples de gélifiants hydrophiles, on peut citer en particulier les polymères carboxyvinyliques (carbomer), les copolymères acryliques tels que les copolymères d'acrylates/alkylacrylates, les polyacrylamides comme par exemple les copolymères réticulés vendus sous les noms Sepigel 305 (nom C.T.F.A. : polyacrylamide/C13-14 isoparaffin/Laureth 7) ou Simulgel 600 (nom C.T.F.A. : acrylamide/sodium acryloyldimethyltaurate copolymer/isohexadecane/polysorbate 80) par la Société SEPPIC ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la Société HOECHST sous la dénomination commerciale « Hostacerin AMPS » (nom C.T.F.A. : ammonium polyacryldimethyltauramide) ; les polysaccharides, les gommes naturelles comme la gomme de xanthane, les argiles, et leurs mélanges.

La composition, lorsqu'elle est fluide, peut contenir éventuellement une phase huileuse.

Cette phase huileuse comporte de préférence au moins une huile.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- des huiles essentielles naturelles ou synthétiques telles que, par exemple, les huiles d'eucalyptus, de lavandin, de lavande, de vétiver, de litsea cubeba, de citron, de santal, de romarin, de camomille, de sarriette, de noix de muscade, de cannelle, d'hysope, de carvi, d'orange, de géraniol, de cade et de bergamote ;
- les alcools gras ayant de 8 à 26 atomes de carbone, comme l'alcool cétylique, l'alcool stéarylique et leur mélange (alcool cétylstéarylique), l'octyl dodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphénylsiloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les gommes telles que les gommes de silicone (diméthiconol) ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Quand elle est présente, la quantité de phase huileuse peut aller par exemple de 0,01 à 50 % en poids et de préférence de 0,1 à 30 % en poids par rapport au poids total de la composition.

La composition selon l'invention peut constituer un gel de bain, des perles de bain, une poudre pour le bain, un lait, une crème de bain, etc..

A partir de la composition selon l'invention, c'est-à-dire d'une composition susceptible de mousser, mais qui ne mousse pas notamment dans le récipient qui la contient, lors du stockage, on obtient une mousse, c'est-à-dire une dispersion de bulles de gaz dans une phase continue aqueuse en exerçant une action mécanique, telle qu'une agitation, sur ladite composition, ou plutôt sur le bain, dans lequel a été ajoutée la composition selon l'invention, qu'elle soit fluide ou solide.

Cette action mécanique peut être, notamment due à l'agitation du bain par l'utilisateur et/ou par un jet ou courant d'eau, ou par tout autre moyen permettant de mélanger la composition ou le volume du bain avec un courant de gaz pour former des bulles de ce gaz dans la phase aqueuse. Le gaz inclus dans la mousse est donc généralement de l'air.

Les compositions de bain selon l'invention peuvent par exemple être utilisées de la manière suivante :
- on introduit la composition selon l'invention dans un volume d'eau destiné à former le bain, cette masse ou volume d'eau peut se trouver déjà à une température « élevée » supérieure à la température de gélification ou bien à une température inférieure ;
- on agite l'ensemble du volume d'eau de manière à répartir de manière homogène la composition de l'invention dans le volume d'eau.

Dans le cas où la température de l'eau est suffisamment élevée, il se formera alors immédiatement un gel-mousse, la mousse se développant du fait de l'agitation. Si le gel ne se forme pas, il est nécessaire d'ajouter à la masse d'eau une quantité supplémentaire de composition selon l'invention pour se trouver dans les plages de concentration où la gélification se produit. Si la température de l'eau n'est pas suffisante, on pourra, par addition d'eau, par exemple d'eau très chaude, faire en sorte que la température du bain dépasse la température de gélification pour donner la texture gélifiée voulue, en prenant soin d'ajouter la quantité suffisante de composition selon l'invention dans le volume du bain.

La température du bain gélifiée sera donc généralement de 30 à 50°C.

L'utilisateur peut se trouver dans le bain préalablement ou postérieurement à l'introduction de la composition de l'invention dans celui-ci.

Peu à peu, la température du bain va proprement diminuer et passer, par exemple, d'une température de 30 à 50°C, qui est celle du bain gélifiée, à une température de 5 à 30°C, à laquelle le bain est fluidifié, et a une viscosité très réduite, du fait de la disparition de l'effet de thermogélification réversible du polymère ou composé inclus dans la composition de bain selon l'invention. Cette diminution de température se produit en une durée de temps, qui correspond généralement à la durée d'un bain.

On peut, bien sûr, accélérer ce refroidissement et la fluidification concommittante du bain en rajoutant de l'eau relativement plus froide au bain, de manière à en abaisser la température jusque dans les plages ou l'effet thermogélifiant, n'existe plus.

Une fois que le bain est redevenu fluide, avec une viscosité faible, il peut être évacué facilement du récipient, tel qu'une baignoire, dans laquelle il se trouve, généralement sous l'effet de la seule gravité ou avec l'assistance d'un rinçage à l'eau froide.

D'autres caractéristiques et avantages de l'invention apparaîtront mieux à la lecture de la description qui suit, donnée à titre illustratif et non limitatif.

### Description détaillée des modes de réalisation

Les exemples suivants illustrent la réalisation de compositions gélifiantes selon l'invention comprenant les polymères préférés comportant des unités hydrosolubles et des unités à LCST spécifiques.

Les polymères utilisés dans ces exemples sont constitués par un squelette d'acide polyacrylique (PAA) portant des chaînes latérales ou greffons constituées par des unités à LCST spécifiques. Ils sont caractérisés par la masse molaire du squelette hydrosoluble (acide polyacrylique), la nature chimique des chaînes à LCST, leur proportion massique dans le polymère et leur masse molaire.

Les caractéristiques des polymères utilisés sont données dans le tableau I.

**Tableau I**

| | Squelette hydrosoluble | Greffons (unités à LCST) | Proportion : unités à LCST dans polymère final (en poids) | Taux de greffage (% en mole) |
|---|---|---|---|---|
| Polymère 1 | Acide polyacrylique; PM=450 000 | (OE)₆(OP)₃₉ statistique Jeffamine M-2005; PM=2 600 | 51 % | 3,9% |
| Polymère 2 | Acide polyacrylique; PM=550 000 | PolyN-isopropylacrylamide (pNIPAM) PM= 10 000 | 49 % | 0,9% |
| Polymère 3 | Acide polyacrylique; PM=450 000 | (OE)₆(OP)₃₉ statistique Jeffamine M-2005 ; PM=2 600 | 59 % | 5,3 % |

Ces polymères sont préparés de la façon suivante.

### Préparation du Polymère 1

Dans un réacteur de 500 ml muni d'un réfrigérant, on dissout 3 g d'acide polyacrylique de masse molaire moyenne 450 000 g/mole (Aldrich) dans 220 ml de N-méthyl pyrrolidone sous agitation à 60°C pendant 12 h.

On dissout 4,181 g de copolymère (OE)₆(OP)₃₉ statistique monoaminé, de masse molaire 2600 g/mole ayant un point de trouble, à une concentration de 1% en poids dans l'eau, de 16°C (Jeffamine M-2005 de Huntsman) dans 50 ml de N-méthylpyrrolidone sous agitation, à 20°C, pendant 15 min.. La solution obtenue est ajoutée goutte à goutte au milieu réactionnel contenant l'acide polyacrylique, sous vive agitation à 60°C.

On dissout 2,158 g de dicyclohexylcarbodiimide dans 30 ml de N-méthylpyrrolidone sous agitation à 20°C pendant 15 min.. La solution obtenue est ajoutée goutte à goutte au milieu réactionnel contenant l'acide polyacrylique et le copolymère (OE)₆(OP)₃₉ statistique monoaminé, sous vive agitation à 60°C. On agite le mélange final pendant 12 h à 60°C.

On refroidit le mélange à 20°C puis on le place dans un réfrigérateur à 4°C pendant 24 h. Les cristaux de dicyclohexylurée formés sont éliminés par filtration du milieu réactionnel.

Le polymère est alors neutralisé à l'aide de 19 g de soude à 35 % (4 fois en excès par rapport au nombre de mole d'acide acrylique), ce qui conduit à sa précipitation. Après 12 h au repos, le milieu réactionnel est filtré afin de récupérer le polymère précipité. Celui-ci est séché sous vide à 35°C pendant 24 h.

On récupère 13,55 g de solide qui sont dissous dans 2 l d'eau désionisée. Cette solution est ultrafiltrée à l'aide d'un système d'ultrafiltration Millipore contenant une membrane dont le seuil de coupure est fixée à 10 000 Daltons. La solution ainsi purifiée est lyophilisée afin de recueillir le polymère sous forme solide.

On obtient 7,05 g de polyacrylate de sodium (450 000 g/mole) greffé par 3,9 % (en mole) de copolymère (OE)₆(OP)₃₉ statistique monoaminé.

La proportion massiques des unités à LCST dans le polymère final est de 51 %.

Le polymère ainsi obtenu présente une solubilité dans l'eau, à 20°C, d'au moins 10 g/l.

### Préparation du polymère 2

Le polymère 2 qui comporte des greffons de poly-N-isopropylacrylamide (pNIPAM) est préparé par un procédé en 2 étapes :

### 1) Synthèse d'oligomères pNIPAM portant une extrémité réactive aminée

Dans un ballon tricol de 250 ml muni d'un réfrigérant et d'une arrivée d'azote sont introduits 8 g de N-isopropylacrylamide et 80 ml de diméthylsulfoxyde. Ce mélange est chauffé sous agitation à 29°C à l'aide d'un bain-marie et placé sous un barbotage d'azote. Après 45 min., 0,161 g de chlorhydrate d'aminoéthanethiol préalablement dissous dans 4 ml de diméthylsulfoxyde sont ajoutés au milieu réactionnel. 5 min. après, 0,191 g de persulfate de potassium dissous dans 8 ml de diméthylsulfoxyde sont ajoutés au milieu réactionnel. Ce milieu réactionnel est maintenu sous agitation et atmosphère d'azote pendant 3 h à 29°C.

Les oligomères de poly N-isopropylacrylamide (pNIPAM) synthétisés sont isolés par précipitation du milieu réactionnel dans un mélange d'acétone (40 % en volume) et d'hexane (60 %).

### 2) Greffage des oligomères de pNIPAM sur de l'acide polyacrylique

Dans un ballon tricol de 250 ml muni d'un réfrigérant sont dissous dans 100 ml de 1-méthyl 2-pyrrolydone 3 g d'acide polyacrylique de masse molaire 550 000 g/mole, sous agitation à 60°C pendant 12 h. 3,757 g d'oligomères pNIPAM préalablement dissous dans 25 ml de 1-méthyl 2-pyrrolidone sont introduits goutte à goutte dans le milieu réactionnel sous agitation. 15 min. après, 0,776 g de dicyclohexylcarbodiimide préalablement dissous dans 25 ml de 1-méthyl 2-pyrrolidone sont introduits goutte à goutte dans le milieu réactionnel sous vive agitation. Le milieu réactionnel est maintenu pendant 12 h à 60°C sous agitation.

Le milieu réactionnel est alors refroidi à 20°C puis placé dans un réfrigérateur à 4°C pendant 24 h. Les cristaux de dicyclohexylurée formés sont alors éliminés par filtration. Le polymère est alors neutralisé à l'aide de 19 g de soude à 35 % (4 fois en excès par rapport au nombre de mole d'acide acrylique), ce qui conduit à sa précipitation. Après 12 h au repos, le milieu réactionnel est filtré afin de récupérer le polymère précipité. Celui-ci est séché sous vide à 35°C pendant 24 h.

On récupère 10,2 g de solide qui sont dissous dans 2 litres d'eau désionisée. Cette solution est ultrafiltrée à l'aide d'un système d'ultrafiltration Millipore contenant une membrane dont le seuil de coupure est fixée à 10 000 Daltons. La solution ainsi purifiée est lyophilisée afin de recueillir le polymère sous forme solide.

On obtient 4,8 g de polyacrylate de sodium (450 000 g/mole) greffé par 0,9% (en mole) de poly N-isopropylacrylamide.

La proportion massique des unités à LCST dans le polymère final est de 49%.

De manière similaire à l'exemple 1, on a préparé le polymère 3, dans lequel les greffons qui sont les mêmes que dans le polymère 1, sont issus d'un copolymère (OE)₆(OP)₃₉ statistique monoaminé (JEFFAMINE M-2005).

On détermine les concentrations d'agrégation critiques (CAC) des trois polymères préparés plus haut, par rhéologie, selon la méthode décrite précédemment, à l'aide d'un rhéomètre Haake RS 150, équipé d'une géométrie cône/plan (35 mm, 2°) et d'un bain thermostaté, afin de contrôler la température entre 5 et 80°C. Les mesures ont été effectuées dans le mode écoulement à une vitesse de cisaillement de 10 s⁻¹, en faisant varier la température de 15 à 50°C à une vitesse de 0,5°C/minute.

Pour le polymère 1 dans NaCl 0,2 M ; la concentration d'agrégation critique (CAC) est d'environ 1 % en poids.

Pour le polymère 2 dans l'eau pure, la concentration d'agrégation critique est d'environ 0,3 % en poids.

Tandis que pour le polymère 3 dans NaCl 0,2 M, la concentration d'agrégation critique (CAC) est d'environ 1 % en poids.

Les mesures rhéologiques effectuées dans les exemples suivants ont été effectuées de la même manière et dans les mêmes conditions que celles réalisées pour déterminer les CAC, mais en faisant varier la température dans la plage de 20 à 35°C, à une vitesse de 0,5°C/min.

### Exemple 1

Cet exemple décrit une composition pour le bain comprenant 5 % en poids du polymère 1.

### a) Composition (il ne s'agit, en fait, pas de la composition de l'invention, mais en fait du bain final préparé par incorporation de la composition de l'invention (qui dans ce cas ne comprend que le polymère et du sel) dans de l'eau)

| | |
|---|---|
| Polymère 1 | 5 g |
| NaCl | 1,17 g |
| Eau déminéralisée | 93,83 g |

Cette composition est préparée par simple introduction du polymère dans l'eau salée sous agitation pendant 2 h.

### b) Propriétés gélifiantes et élimination du bain par refroidissement

Les propriétés gélifiantes de la composition préparée ci-dessus, et sa fluidification par refroidissement ont été mises en évidence par des mesures rhéologiques.

La composition de bain obtenue selon l'invention présente les viscosités suivantes.

Viscosité à 20°C (10s⁻¹) = 0,015 Pa.s.

Viscosité à 35°C (10s⁻¹) = 10 Pa.s.

Viscosité à 20°C, après chauffage à 35°C, (10s⁻¹) : 0,015 Pa.s.

Le pouvoir gélifiant du polymère 1 permet d'obtenir un bain gélifié à 35°C.

Lorsque la température diminue de 35°C à 20°C, la composition se fluidifie et peut ainsi être facilement éliminée.

### Exemple 2

Cet exemple décrit une composition pour le bain comprenant 1 % en poids du polymère 2.

### a) Composition

| | |
|---|---|
| Polymère 2 | 1 g |
| Eau déminéralisée | 99 g |

Cette composition est préparée par simple introduction du polymère dans l'eau sous agitation pendant 2 h.

### b) Propriétés gélifiantes et élimination du bain par refroidissement

Les propriétés gélifiantes de la composition préparée ci-dessus et sa fluidification par refroidissement ont été mises en évidence par des mesures rhéologiques.

La composition de bain obtenue selon l'invention présente les viscosités suivantes.

Viscosité à 20°C (10s⁻¹) = 0,17 Pa.s.

Viscosité à 35°C (10s⁻¹) = 0,7 Pa.s.

Viscosité à 20°C, après chauffage à 35°C, (10s-1) : 0,16 Pa.s.

Le pouvoir gélifiant du polymère 2 permet d'obtenir un bain gélifié à 35°C.

Lorsque la température diminue de 35°C à 20°C, la composition se fluidifie et peut ainsi être facilement éliminée.

### Exemple 3

Cet exemple décrit une composition pour le bain comprenant 5 % en poids du polymère 3 et 3 % d'un tensioactif alkyl polyglycoside.

Le tensioactif utilisé est un alkyl C₁₀₋₁₄ polyglycoside, commercialisé sous le nom de ORAMIX® NS10 par SEPPIC®.

### a) Composition

| | |
|---|---|
| Polymère 3 | 5 g |
| ORAMIX® NS10 | 3 g |
| Eau déminéralisée | 92 g |

Cette composition est préparée par mélange en proportions égales d'une solution à 10 % du polymère 3 et d'une solution à 6 % d'ORAMIX® NS10. Ces deux solutions mères sont préparées par simple introduction du polymère (ou du tensioactif) dans l'eau, sous agitation, pendant 2 h.

### b) Propriétés gélifiantes et élimination du bain par refroidissement

Les propriétés gélifiantes de la composition préparée ci-dessus et sa fluidification par refroidissement ont été mises en évidence par des mesures rhéologiques.

La composition de l'invention présente les viscosités suivantes.

Viscosité à 20°C (10s⁻¹) = 0,06 Pa.s.

Viscosité à 35°C (10s⁻¹) = 0,8 Pa.s.

Viscosité à 20°C, après chauffage à 35°C, (10s⁻¹) : 0,07 Pa.s.

Le pouvoir gélifiant du polymère 3 permet d'obtenir un bain gélifié à 35°C.

Lorsque la température diminue de 35°C à 20°C, la composition se fluidifie et peut ainsi être facilement éliminée.

### Exemple 4

Cet exemple décrit un système thermogélifiant et une composition pour le bain comprenant un polymère associatif associé à un tensioactif formant des structures en bicouche en solution aqueuse.

### a) Système thermogélifiant

Polyacrylate de sodium de masse molaire 150 000 g/mole, greffé par 3 % (en mole) de chaînes dodécyle, mis en présence du tensioactif alkyléthoxylé C₁₂(OE)₄.

### b) Composition du bain

| | |
|---|---|
| Polymère | 1 g |
| C₁₂(OE)₄ | 3,24 g |
| Eau déminéralisée | 95,76 g |

Cette composition est préparée par simple mélange d'une solution à 2 % du polymère et d'une solution à 6,48 % du tensioactif. Ces deux solutions mères sont préparées par simple introduction du polymère (ou du tensioactif) dans l'eau sous agitation.

### c) Propriétés gélifiantes et élimination du bain par refroidissement

Les propriétés gélifiantes de la composition préparée ci-dessus et sa fluidification par refroidissement ont été mises en évidence par des mesures rhéologiques.

La composition de l'invention présente les viscosités suivantes :

| | |
|---|---|
| Viscosité à 20°C (10 s⁻¹) | 1 Pa.s |
| Viscosité à 35°C (10 s⁻¹) | 40 Pa.s |
| Viscosité à 20°C, après chauffage à 35°C (10⁻¹) | 1 Pa.s |

Le pouvoir gélifiant du système polymère + tensioactif permet d'obtenir un bain gélifié à 35°C. Lorsque la température diminue de 35°C à 20°C, la composition se fluidifie et peut facilement être éliminée.

### Exemple 5

Cet exemple décrit un système gélifiant et une composition pour le bain comprenant de la méthyl cellulose.

### a) Système thermogélifiant

METHOCEL® A100 fourni par Dow Chemical ; son degré de substitution en groupes méthoxy est compris entre 1,6 et 1,8.

### b) Composition du bain

| | |
|---|---|
| Polymère | 5 g |
| Eau déminéralisée | 95 g |

Cette composition est préparée par simple introduction du polymère dans l'eau sous agitation.

### c) Propriétés gélifiantes et élimination du bain par refroidissement

Les propriétés gélifiantes de la composition préparée ci-dessus et sa fluidification par refroidissement ont été mises en évidence par des mesures rhéologiques.

La composition de l'invention présente les viscosités suivantes :

| | |
|---|---|
| Viscosité à 20°C (10 s⁻¹) | 1 Pa.s |
| Viscosité à 40°C (10 s⁻¹) | 10 Pa.s |
| Viscosité à 20°C, après chauffage à 40°C (10 s⁻¹) | 0,9 Pa.s |

Le pouvoir gélifiant de ce polymère permet d'obtenir un bain gélifié à 40°C. Lorsque la température diminue de 40°C à 20°C, la composition se fluidifie et peut facilement être éliminée.

### Exemple 6

Cet exemple décrit un système thermogélifiant et une composition pour le bain comprenant un copolymère bloc POE/POP/POE.

### a) Système thermogélifiant

Pluronic P-85 fourni par BASF ; sa masse molaire est de 4 500 g/mole ; la masse molaire des blocs POE est égale à 1 125 g/mole et celle du bloc POP est égale à 2 250 g/mole.

### b) Composition du bain

| | |
|---|---|
| Polymère | 27 g |
| Eau déminéralisée | 73 g |

Cette composition est préparée par simple introduction du polymère dans l'eau sous agitation.

### c) Propriétés gélifiantes et élimination du bain par refroidissement

Les propriétés gélifiantes de la composition préparée ci-dessus et sa fluidification par refroidissement ont été mises en évidence par des mesures rhéologiques.

La composition de l'invention présente les viscosités suivantes :

| | |
|---|---|
| Viscosité à 20°C (10 s⁻¹) | 0,03 Pa.s |
| Viscosité à 35°C (10 s⁻¹) | 150 Pa.s |
| Viscosité à 20°C, après chauffage à 35°C (10 s⁻¹) | 0,04 Pa.s |

Le pouvoir gélifiant de ce polymère permet d'obtenir un bain gélifié à 35°C. Lorsque la température diminue à 35°C à 20°C, la composition se fluidifie et peut facilement être éliminée.

### REFERENCES

[1] N. SARKAR, J. Appl. Polym. Sci., 1979, 24, 1 073.
[2] W. BROWN, K. SCHILLEN, M. ALMGREN, S. HVIDT, P. BAHADUR, J. Phys. Chem., 1991, 95, 1 850.
[3] A. CARLSSON, G. KARLSTROM, B. LINDMAN, Coll. Surf. 1990, 47, 147.
[4] K. LOYEN, I. ILIOPOULOS, R. AUDEBERT, Langmuir, 1994, 10, 1 421.
[5] D. HOURDET et al., Polymer, 1994, vol. 35, n° 12, pages 2 624-2 630.
[6] F. L'ALLORET et al., Coll. Polym. Sci., 1995, vol. 273, pages 1 163-1 173.
[7] F. L'ALLORET, Revue de l'Institut Français du Pétrole, 1997, vol. 52, n° 2, pages 117-128.
[8] EP-A-0 583 814.
[9] EP-A-0 629 649.
[10] WO-A-95 24430.
[11] US-A-5 939 485.
[12] WO-A-97 00275.
[13] WO-A-98 48768.
[14] WO-A-00 35961.
[15] Articles de TAYLOR et al., Journal of Polymer Science, part A : Polymer Chemistry, 1975, 13, 2 551.
[16] J. BAILEY et al., Journal of Applied Polymer Science, 1959, 1,56.
[17] HESKINS et al., Journal of Macromolecular Science, Chemistry A2, 1968, 1 441.
[18] EP-A-0 566 438.
[19] FR-A-2 739 556.

## Revendications

1. Composition de bain gélifiante, comprenant au moins un polymère ou composé gélifiant, ledit polymère ou composé gélifiant étant un polymère ou composé thermogélifiant dont les propriétés de gélification par chauffage sont réversibles avec la température.

2. Composition selon la revendication 1, dans laquelle ledit polymère ou composé thermogélifiant est choisi parmi les composés ou polymères suivants :
- les polymères hydrosolubles comprenant des unités hydrosolubles et des unités présentant dans l'eau une température inférieure critique de démixtion (LCST) ;
- les dérivés cellulosiques, tels que la méthylcellulose et l'hydroxypropylméthylcellulose ;
- les copolymères à blocs de type polyoxyalkylénés ;
- les éthers de cellulose non ioniques ;
- les polymères hydrosolubles associatifs en présence d'au moins un agent tensioactif, formant des structures en bicouches en solution aqueuse.

3. Composition de bain selon la revendication 2, dans laquelle ledit polymère ou composé thermogélifiant est un polymère hydrosoluble comprenant des unités hydrosolubles et des unités présentant dans l'eau une température inférieure critique de démixtion LCST.

4. Composition selon la revendication 3, dans laquelle la température de démixtion par chauffage en solution aqueuse desdites unités à LCST du polymère est de 5 à 50°C pour une concentration massique dans l'eau de 1 % desdites unités.

5. Composition de bain selon la revendication 3, dans laquelle la température de démixtion par chauffage en solution aqueuse des unités à LCST du polymère est de 15 à 40°C pour une concentration massique dans l'eau de 1 % desdites unités.

6. Composition de bain selon la revendication 3, dans laquelle le polymère se présente sous la forme d'un polymère séquencé (ou blocs) comprenant des séquences constituées d'unités hydrosolubles alternées avec des séquences constituées d'unités à LCST, ou sous la forme d'un polymère greffé dont le squelette est formé d'unités hydrosolubles ledit squelette étant porteur de greffons constitués d'unités à LCST, lesdits polymères pouvant être partiellement réticulés.

7. Composition de bain selon la revendication 3, dans laquelle les unités hydrosolubles sont, en totalité ou en partie, susceptibles d'être obtenues par polymérisation, ou par polycondensation ou encore sont constituées, en totalité ou en partie, par des polymères naturels ou naturels modifiés.

8. Composition de bain selon la revendication 7, dans laquelle les unités hydrosolubles sont, en totalité ou en partie, susceptibles d'être obtenues par polymérisation, notamment radicalaire, d'au moins un monomère choisi parmi les monomères suivants :
1) l'acide (méth)acrylique ;
2) les monomères vinyliques de formule (I) suivante : 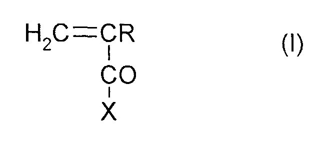 dans laquelle :
- R est choisi parmi H, -CH₃, -C₂H₅ ou -C₃H₇, et
- X est choisi parmi :
- les oxydes d'alkyle de type -OR' où R' est un radical hydrocarboné, linéaire ou ramifié, saturé ou insaturé, ayant de 1 à 6 atomes de carbone, éventuellement substitué par au moins un atome d'halogène (iode, brome, chlore, fluor); un groupement sulfonique (-SO₃⁻), sulfate (-SO₄⁻), phosphate (-PO₄H₂) ; hydroxy (-OH) ; amine primaire (-NH₂) ; amine secondaire (-NHR₁), tertiaire (-NR₁R₂) ou quaternaire (-N⁺R₁R₂R₃) avec R₁, R₂ et R₃ étant, indépendamment l'un de l'autre, un radical hydrocarboné, linéaire ou ramifié, saturé ou insaturé ayant 1 à 6 atomes de carbone, sous réserve que la somme des atomes de carbone de R' + R₁ + R₂ + R₃ ne dépasse pas 7 ; et
- les groupements -NH₂, -NHR₄ et -NR₄R₅ dans lesquels R₄ et R₅ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₄ + R₅ ne dépasse pas 7, lesdits R₄ et R₅ étant éventuellement substitués par un atome d'halogène (iode, brome, chlore, fluor) ; un groupement hydroxy (-OH) ; sulfonique (-SO₃⁻) ; sulfate (-SO₄⁻) ; phosphate (-PO₄H₂) ; amine primaire (-NH₂) ; amine secondaire (NHR₁), tertiaire (-NR₁R₂) et/ou quaternaire (-N⁺R₁R₂R₃) avec R₁, R₂ et R₃ étant, indépendamment l'un de l'autre, un radical hydrocarboné, linéaire ou ramifié, saturé ou insaturé ayant 1 à 6 atomes de carbone, sous réserve que la somme des atomes de carbone de R₄ + R₅ + R₁ + R₂ + R₃ ne dépasse pas 7 ;
3) l'anhydride maléique ;
4) l'acide itaconique ;
5) l'alcool vinylique de formule CH₂=CHOH ;
6) l'acétate de vinyle de formule CH₂=CH-OCOCH₃ ;
7) les N-vinyllactames tels que la N-vinylpyrrolidone, la N-vinylcaprolactame et la N-butyrolactame ;
8) les vinyléthers de formule CH₂=CHOR₆ dans laquelle R₆ est un radical hydrocarboné, linéaire ou ramifié, saturé ou insaturé, ayant de 1 à 6 atomes de carbone ;
9) les dérivés hydrosolubles du styrène, notamment le styrène sulfonate ;
10) le chlorure de diméthyldiallyl ammonium ; et
11) la vinylacétamide.

9. Composition selon la revendication 8, dans laquelle le monomère de formule (I) est choisi parmi l'acide 2-acrylamido-2-méthylpropane sulfonique (AMPS) et l'acide (méth)acrylique.

10. Composition de bain selon la revendication 7, dans laquelle les unités hydrosolubles du polymère sont constituées en totalité ou en partie par des polycondensats ou par des polymères naturels ou naturels modifiés choisis parmi un ou plusieurs des composants suivants :
- les polyuréthannes hydrosolubles ;
- la gomme de xanthane ;
- les alginates et leurs dérivés tels que l'alginate de propylèneglycol ;
- les dérivés de cellulose et notamment la carboxyméthylcellulose, l'hydroxypropylcellulose, l'hydroxyéthylcellulose et l'hydroxyéthylcellulose quaternisée ;
- les galactomannanes et leurs dérivés tels que la gomme Konjac, la gomme de guar, l'hydroxypropylguar, l'hydroxypropylguar modifié par des groupements méthylcarboxylate de sodium, et le chlorure de guar hydroxypropyl triméthyl ammonium ; et
- la polyéthylène-imine.

11. Composition de bain selon l'une quelconque des revendications 7 à 10, dans laquelle les unités hydrosolubles du polymère ont une masse molaire allant de 1 000 g/mole à 50 000 000 g/mole lorsqu'elles constituent le squelette hydrosoluble d'un polymère greffé, ou une masse molaire allant de 500 g/mole à 100 000 g/mole lorsqu'elles constituent un bloc d'un polymère multiblocs.

12. Composition de bain selon l'une quelconque des revendications 3 à 11, dans laquelle les unités à LCST du polymère sont constituées d'un ou plusieurs polymères choisis parmi les polymères suivants :
- les polyéthers tels que le polyoxyde d'éthylène (POE), le polyoxyde de propylène (POP), et les copolymères statistiques d'oxyde d'éthylène (OE) et d'oxyde de propylène (OP) ;
- les polyvinylméthyléthers ;
- les dérivés polymériques et copolymériques N-substitués de l'acrylamide ayant une LCST ; et
- le poly-N-vinylcaprolactame et les copolymères de N-vinyl caprolactame.

13. Composition de bain selon l'une quelconque des revendications 3 à 12, dans laquelle les unités à LCST du polymère sont constituées de poly oxyde de propylène (OP)ₙ où n est un nombre entier de 10 à 50, ou de copolymères statistiques d'oxyde d'éthylène (OE) et d'oxyde de propylène (OP), représentés par la formule :
(OE)ₘ (OP)ₙ
dans laquelle m est un nombre entier allant de 1 à 40, de préférence de 2 à 20, et n est un nombre entier allant de 10 à 60, de préférence de 20 à 50.

14. Composition de bain selon la revendication 13, dans laquelle la masse molaire des unités à LCST du polymère est de 500 à 5 300 g/mole, de préférence de 1 500 à 4 000 g/mole.

15. Composition de bain selon l'une quelconque des revendications 3 à 13, dans laquelle les unités à LCST du polymère sont constituées par un polymère choisi parmi le poly N-isopropylacrylamide, le poly N-éthyl acrylamide et les copolymères de N-isopropylacrylamide ou de N-éthylacrylamide et d'un monomère vinylique choisi parmi les monomères ayant la formule (I) donnée dans la revendication 8, l'anhydride maléique, l'acide itaconique, la vinylpyrrolidone, le styrène et ses dérivés, le chlorure de diméthyldiallyl ammonium, la vinylacétamide, l'alcool vinylique, l'acétate de vinyle, les vinyléthers et les dérivés de l'acétate de vinyle.

16. Composition de bain selon la revendication 15, dans laquelle la masse molaire des unités à LCST du polymère est de 1 000 g/mole à 500 000 g/mole, de préférence de 2 000 à 50 000 g/mole.

17. Composition de bain selon l'une quelconque des revendications 3 à 12, dans lequel les unités à LCST du polymère sont constituées par un poly-N-vinylcaprolactame ou un copolymère de N-vinylcaprolactame et d'un monomère vinylique choisi parmi les monomères répondant à la formule (I) donnée dans la revendication 8, l'anhydride maléique, l'acide itaconique, la vinylpyrrolidone, le styrène et ses dérivés, le chlorure de diméthyldiallyl ammonium, la vinylacétamide, l'alcool vinylique, l'acétate de vinyle, les vinyléthers et les dérivés de l'acétate de vinyle.

18. Composition de bain selon la revendication 17, dans lequel la masse molaire des unités à LCST est de 1 000 à 500 000 g/mole, de préférence de 2 000 à 50 000 g/mole.

19. Composition de bain selon l'une quelconque des revendications 3 à 18, dans laquelle la proportion massique des unités à LCST du polymère est de 5 à 70 %, de préférence de 20 à 65 % et mieux encore de 30 à 60 %, par rapport au polymère.

20. Composition de bain selon l'une quelconque des revendications 1 à 19, qui est sous forme solide.

21. Composition de bain selon la revendication 20, qui est une composition en particules.

22. Composition de bain selon la revendication 20 ou la revendication 21, dans laquelle la concentration massique du polymère ou composé thermogélifiant est de 0,5 à 1 g par gramme de composition solide.

23. Composition de bain selon l'une quelconque des revendications 1 à 19 qui est une composition liquide comportant essentiellement une phase aqueuse continue.

24. Composition de bain selon la revendication 23, dans laquelle ladite phase aqueuse continue se présente sous la forme d'une solution aqueuse concentrée en polymère ou composé.

25. Composition de bain fluide selon la revendication 23 ou 24, dans laquelle la concentration massique en polymère ou composé de la phase aqueuse est de 0,1 à 90 %.

26. Composition selon l'une quelconque des revendications 1 à 25, qui est une composition moussante.

27. Composition de bain selon l'une quelconque des revendications 1 à 26, contenant, en outre, au moins un agent tensioactif moussant.

28. Composition selon la revendication 27, dans laquelle ledit agent tensioactif moussant est non ionique.

29. Composition selon l'une quelconque des revendications 1 à 28, dans laquelle la phase aqueuse ou la composition solide est constituée par un milieu physiologiquement acceptable permettant une application topique et notamment cosmétique.

30. Utilisation du polymère ou composé tel que décrit dans l'une quelconque des revendications 1 à 8, pour gélifier un volume ou une masse d'eau à une température déterminée dans un récipient, et fluidifier ledit volume d'eau lors de son refroidissement, afin de faciliter l'évacuation dudit récipient.
